# EUROPEAN PATENT SPECIFICATION

(11) **EP 3 946 532 B1**
(45) Date of publication and mention of the grant of the patent: **02.09.2026**
(21) Application number: 20783440.9
(22) Date of filing: 27.03.2020
(51) Int. Cl.: A61M 16/00, A61M 16/20, F01N 1/02

(54) **MUFFLER**
SCHALLDÄMPFER
SILENCIEUX

(30) Priority: 29.03.2019 US 201962826474 P
(43) Date of publication of application: 09.02.2022
(73) Proprietor: Fisher & Paykel Healthcare Limited, East Tamaki Auckland 2013 (NZ)
(72) Inventor: PROLE, Dylan Peter, Auckland, 2013 (NZ); MASSEY, Shane Terry, Auckland, 2013 (NZ); EDWARDS, Taylor James, Auckland, 2013 (NZ); KAT, Arjen David, Auckland, 2013 (NZ); OSBORNE, Hamish Adrian, Auckland, 2013 (NZ)
(74) Representative: Ström & Gulliksson AB
(86) International application number: PCT/IB2020/052894
(87) International publication number: WO 2020/201947

(56) References cited:
- WO-A1-2017/027906
- WO-A1-2018/033863
- WO-A1-2018/085889
- WO-A2-2012/103526
- CN-A- 108 159 540
- CN-U- 201 934 791
- US-A- 4 220 219
- US-A1- 2004 035 422
- US-A1- 2008 127 976
- US-A1- 2008 127 976
- US-A1- 2008 269 669
- US-A1- 2010 078 017
- US-A1- 2013 199 538
- US-A1- 2013 199 538
- US-A1- 2014 299 132
- US-A1- 2018 185 597
- US-B1- 6 615 831
- US-B2- 7 506 722
- US-B2- 7 527 053

## Description

### BACKGROUND

### Field of the invention

The present disclosure generally relates to a pressure relief valve comprising a muffler for use in respiratory support systems. The muffler comprises one or more structures that are arranged to provide a gas flow path having variable volumes or areas and/or to provide a tortuous gas flow path through the muffler.

### Description of the Related Art

Respiratory gas supply systems provide gas for delivery to a patient. Respiratory gas supply systems typically include a fluid connection between the gas supply and the patient. This may include a gas delivery conduit, such as an inspiratory tube that is connected to a patient interface. Such systems may be open, i.e. comprising an unsealed patient interface such as a nasal cannula, or closed, i.e. comprising a sealed patient interface such as a face mask that seals against the user's face. Such systems may receive gases from a pressurised gas supply (such as a gas tank, or hospital wall supply), a blower, or a combination thereof.

Open respiratory gas supply systems may include those employed in nasal high flow therapy, for example. Closed respiratory gas supply systems may include those employed in continuous positive airway pressure (CPAP) or in ventilation, for example.

It is common for respiratory support systems to be provided to patients in hospitals, especially in surgical theatres. In such situations, the patient receives breathing gas from a respiratory system. The respiratory system typically comprises a patient interface and at least one conduit in fluid communication with a flow source. The respiratory system may also comprise a humidifier to humidify the breathing gas.

The breathing gas is typically provided to the respiratory system from a wall flow source. The wall flow source provides breathing gas at a predetermined pressure range, higher than that of atmospheric pressure. The pressurised compressed gas flowing from the wall source emits a high frequency sound, which is unpleasant for people in the vicinity and, in some countries, may be in breach of sound regulations for surgical theatres. In some cases, the sound emitted is about 70dBA or more.

Respiratory systems used for CPAP or ventilation may also emit a noise as a result of breathing gas passing through the system under pressure. Any noise emitted, particularly high frequency noises, may be disturbing to the user and others in the vicinity, especially if the respiratory system is used at night to provide respiratory support to a person sleeping.

It would be useful to provide a muffler that is configured to be used within such respiratory systems and that attenuates sound or that at least provides the public with a useful alternative to known systems.

WO2018/033863 describes a solution for reducing noise from moving valve components. CN201934791 describes a silencer for attenuating the sound of exhaust gases from a valve. US2008/269669 describes an apparatus for subcutaneous delivery of gas for therapeutic purposes, the apparatus comprising a relief valve and a silencer disposed on an outlet of the relief valve. US2010/078017A1 describes a system for wireless data communication for use with a breathing assistance system configured to provide breathing assistance to a patient.

In this specification where reference has been made to patent specifications, other external documents, or other sources of information, this is generally to provide a context for discussing features of the invention. Unless specifically stated otherwise, reference to such external documents or sources of information is not to be construed as an admission that such documents or such sources of information, in any jurisdiction, are prior art or form part of the common general knowledge in the art.

### SUMMARY OF THE INVENTION

The invention is defined in the appended independent claim 1. Preferred embodiments are matter of the dependent claims.

In a first aspect, the invention provides a pressure relief valve comprising: an inlet and an outlet, and a respiratory system muffler located along a gas flow path of the pressure relief valve, the gas flow path extending between the valve inlet and the valve outlet, wherein the muffler comprises a tortuous gas flow path to attenuate sound, wherein the valve inlet comprises an engagement mechanism to couple the pressure relief valve to a gas flow source and wherein the valve outlet is connectable to a humidifier via a gas conduit to provide fluid communication between the pressure relief valve and the humidifier.

The pressure relief valve comprises a flow compensated pressure relief valve and is configured to allow gases from a flow of gases along the gas flow path to vent above a pressure threshold.

In one form, the muffler is located at the valve inlet or the valve outlet or both.

In one form, the outlet is connectable to a humidifier via a gas conduit to provide fluid communication between the pressure relief valve and the humidifier.

In one form, the tortuous flow path comprises different cross-sectional gas flow areas.

In one form, the tortuous flow path comprises at least one contraction portion where the gas flow is caused to contract and at least one expansion portion where the gas flow is caused to expand.

In one form, the muffler comprises a housing, a muffler inlet, a muffler outlet, and a sound attenuating structure that defines a gap between a peripheral surface of the sound attenuating structure and an internal wall of the housing, wherein the gap forms a portion of the gas flow path.

In one form, the sound attenuating structure comprises a laterally extending projection that extends towards the internal wall of the housing.

In one form, the laterally extending projection terminates proximate to the internal wall of the housing and at least a portion of the gas flow path is defined by a gap formed between the peripheral surface of the projection and the internal wall of the housing.

In one form, the gap is about 0.5 mm wide or less.

In one form, the gap has a width that is between about 0.1 mm to about 0.5 mm inclusive.

In one form, the gap is about 0.25 mm wide.

In one form, the muffler comprises two or more sound attenuating structures.

In one form, an expansion chamber is defined between two adjacent ones of the two or more sound attenuating structures.

In one form, a constant distance is provided between the sound attenuating structures.

In one form, a variable distance is provided between the sound attenuating structures.

In one form, each sound attenuating structure has the same thickness.

In one form, at least one of the sound attenuating structures has a different thickness to one or more others of the sound attenuating structures.

In one form, the muffler inlet comprises a flow directing element that directs gas flow to the sound attenuating structure(s).

In one form, the muffler inlet comprises a flow directing element that directs gas flow to the expansion chamber.

In one form, the muffler inlet comprises one or more inlet apertures.

In one form, the muffler comprises a terminal end plate on which the outlet is located and wherein the outlet comprises one or more outlet apertures.

In one form, the muffler comprises one or more sound absorbing materials.

In one form, the distance from the muffler inlet to the muffler outlet corresponds to a sound frequency to be reduced, removed or dampened by the muffler.

In one form, the distance from the muffler inlet to the muffler outlet is at least 20mm.

In one form, the distance from the muffler inlet to the muffler outlet is between about 20mm to about 100mm inclusive.

In one form, the muffler comprises an outlet end portion, on which the muffler outlet is located, and wherein the outlet end portion comprises a sealing element adapted to seal against a surface of the pressure relief valve.

In one form, the sealing element is located on an external surface of the muffler housing.

In one form, the valve comprises an engagement mechanism to couple the muffler to the pressure relief valve.

In one form, the engagement mechanism comprises screw threads.

In one form, the muffler comprises a muffler according to the second aspect of the disclosure.

In a second aspect, the present disclosure relates to a respiratory system muffler, the muffler comprising: an inlet, an outlet, and a gas flow path extending between the inlet and the outlet and having a variable cross-sectional area; wherein the gas flow path comprises one or more expansion portions comprising a first cross-sectional area and one or more contraction portions comprising a second cross-sectional area; and wherein the first cross-sectional area is generally larger than the second cross-sectional area.

Optionally, the gas flow path comprises alternating expansion and contraction portions.

In one form, the first cross-sectional area is at least two times larger than the second cross-sectional area of the gas flow path.

Optionally, at least one of the expansion portions has a width that is about 10 times to about 20 times greater than a width of at least one of the contraction portions.

In one form, at least one of the expansion portions has a width that is about 10 times to about 15 times greater than a width of at least one of the contraction portions.

In one form, at least one of the contraction portions of the gas flow path has a width of less than about 0.5mm.

In one form, at least one of the contraction portions of the gas flow path has a width of between about 0.1mm to 0.5mm inclusive.

In one form, at least one of the contraction portions of the gas flow path has a width of about 0.25 mm.

In one form, at least one of the expansion portions of the gas flow path has a width of more than about 1.0mm.

In one form, at least one of the expansion portions of the gas flow path has a width of between about 3.0mm to about 4.0mm inclusive.

In one form, at least one of the expansion portions of the gas flow path has a width of about 3.5mm.

In one form, the muffler comprises at least one sound attenuating structure that projects generally laterally toward an internal wall of the muffler and wherein a gap is defined between the sound attenuating structure and the internal wall. In one form, the gap comprises the one or more contraction portions of the gas flow path.

In one form, the muffler comprises a shaft. Optionally, at least one sound attenuating structure projects generally perpendicularly from the shaft. In one form, the sound attenuating structure comprises a generally circular peripheral edge. In one form, at least one channel through the sound attenuating structure.

In one form, the muffler comprises two or more sound attenuating structures.

In one form, an expansion chamber is defined between two adjacent ones of two or more sound attenuating structures, and wherein the chamber comprises an expansion portion of the gas flow path that comprises the first cross-sectional area.

In one form, the inlet comprises a flow directing element. In one form, the flow directing element directs gas flow to at least one of the sound attenuating structures. In another form, the inlet comprises a flow directing element that directs gas flow to at least one expansion chamber.

In one form, a constant distance is provided between the sound attenuating structures.

In one form, a variable distance is provided between the sound attenuating structures.

In one form, each sound attenuating structure has the same thickness.

In one form, at least one of the sound attenuating structures has a different thickness to one or more others of the sound attenuating structures.

In one form, each sound attenuating structure comprises a generally circular outwardly facing peripheral surface.

In one form, the inlet comprises one or more inlet apertures.

In one form, the muffler comprises a terminal end plate in which the outlet is located and wherein the outlet comprises one or more outlet apertures.

In one form, the distance from the inlet to the outlet corresponds to a sound frequency to be reduced, removed or dampened by the muffler.

In one form, the distance from the inlet to the outlet is at least 20mm.

In one form, the distance from the inlet to the outlet is between about 20mm to about 100mm inclusive.

In one form, the muffler comprises one or more sound absorbing materials.

In one form, the muffler comprises an outlet end portion, on which the outlet is located, and wherein the outlet end portion comprises a sealing element adapted to seal against a surface of a respiratory device component, wherein optionally the respiratory device component comprises a pressure relief valve.

In one form, the sealing element is located on an external surface of the muffler.

In one form, the muffler comprises an engagement mechanism to couple the muffler to a respiratory device component, wherein optionally the respiratory device component comprises a pressure relief valve.

In one form, the inlet comprises an engagement mechanism to engage a gas flow source.

In one form, the engagement mechanism comprises screw threads.

Also disclosed herein is a respiratory system muffler comprising: a central longitudinal axis and comprising an inlet, an outlet, and a sound attenuating structure that forms a tortuous gas flow path around the central longitudinal axis of the muffler between the inlet and the outlet.

In one form, a portion of the gas flow path is defined by a gap between the sound attenuating structure and an internal wall of the muffler.

In one form, the gap is about 0.5mm wide.

In one form, the gap has a width of between about 0.1mm to 0.5mm inclusive.

In one form, the gap is about 0.25 mm wide.

In one form, the inlet comprises one or more inlet apertures.

In one form, the muffler comprises a shaft extending along or substantially parallel to the central longitudinal axis and wherein the sound attenuating structure projects generally laterally from the shaft.

In one form, the sound attenuating structure projects perpendicularly from the shaft.

In one form, the muffler comprises two or more sound attenuating structures

In one form, an expansion chamber is defined between two adjacent ones of the two or more sound attenuating structures.

In one form, the gap between each sound attenuating structure and the internal wall comprises a contraction portion of the gas flow path and each expansion chamber comprises an expansion portion of the gas flow path to form a gas flow path comprising alternating contraction and expansion portions.

In one form, the expansion portion of the gas flow path comprises a first cross-sectional area and the contraction portion of the gas flow path comprises a second cross-sectional area and wherein the first cross-sectional area is at least two times larger than the second cross-sectional area of the gas flow path.

In one form, at least one of the expansion portions has a width that is about 10 times to about 20 times greater than a width of at least one of the contraction portions.

In one form, at least one of the expansion portions has a width that is about 10 times to about 15 times greater than a width of at least one of the contraction portions.

In one form, at least one of the expansion portions of the gas flow path has a width of more than about 1.0mm.

In one form, at least one of the expansion portions of the gas flow path has a width of between about 3.0mm to about 4.0mm inclusive.

In one form, at least one of the expansion portions of the gas flow path has a width of about 3.5mm.

In one form, the inlet comprises a flow directing element that directs gas flow to at least one of the sound attenuating structures.

In one form, the inlet comprises a flow directing element that directs gas flow to at least one of the expansion chambers.

In one form, a constant distance is provided between the sound attenuating structures.

In one form, a variable distance is provided between the sound attenuating structures.

In one form, each sound attenuating structure has the same thickness.

In one form, at least one of the sound attenuating structures has a different thickness to one or more others of the sound attenuating structures.

In one form, each sound attenuating structure comprises a generally circular outwardly facing peripheral surface.

In one form, the muffler further comprises a terminal end plate in which the outlet is located and wherein the outlet comprises one or more outlet apertures.

In one form, the distance from the inlet to the outlet corresponds to a sound frequency to be reduced, removed or dampened by the muffler.

In one form, the distance from the inlet to the outlet is at least about 20mm.

In one form, the distance from the inlet to the outlet is between about 20mm to about 100mm inclusive.

In one form, the muffler comprises one or more sound absorbing materials.

In one form, the muffler comprises an outlet end portion, on which the outlet is located, and wherein the outlet end portion comprises a sealing element adapted to seal against a surface of a respiratory device component.

In one form, the sealing element is located on an external surface of the muffler.

In one form, the muffler comprises an engagement mechanism to couple the muffler to a respiratory device component.

In one form, the respiratory system component comprises a pressure relief valve.

In one form, the inlet comprises an engagement mechanism to engage a gas flow source.

In one form, the engagement mechanism comprises screw threads.

Also disclosed herein is a respiratory system muffler comprising: an inlet; an outlet; a housing; and a body receivable within the housing and comprising at least one sound attenuating structure; wherein a tortuous gas flow path extends from the inlet to the outlet and wherein at least a portion of the gas flow path is defined between the sound attenuating structure and the housing.

In one form, the tortuous gas flow path comprises variable cross-sectional gas flow areas.

In one form, the body comprises a core comprising a shaft.

In one form, the core is generally cylindrical.

In one form, the sound attenuating structure comprises a projection that extends from the shaft towards an internal wall of the housing.

In one form, the sound attenuating structure projects generally laterally towards the internal wall of the housing.

In one form, the sound attenuating structure projects perpendicularly from the shaft.

In one form, the sound attenuating structure terminates proximate to the internal wall and at least a portion of the tortuous gas flow path is defined by a gap formed between the sound attenuating structure and the internal wall of the housing.

In one form, the gap is about 0.5mm wide.

In one form, the gap has a width of between about 0.1mm to 0.5mm inclusive.

In one form, the gap is about 0.25 mm wide.

In one form, the inlet comprises one or more inlet apertures.

In one form, the muffler comprises two or more sound attenuating structures.

In one form, an expansion chamber is defined between two adjacent ones of the two or more sound attenuating structures.

In one form, the gap between each sound attenuating structure and the internal wall comprises a contraction portion of the gas flow path and each expansion chamber comprises an expansion portion of the gas flow path to form a gas flow path comprising alternating contraction and expansion portions.

In one form, the expansion portion of the gas flow path comprises a first cross-sectional area and the contraction portion of the gas flow path comprises a second cross-sectional area and wherein the first cross-sectional area is at least two times larger than the second cross-sectional area of the gas flow path.

In one form, at least one of the expansion portions has a width that is about 10 times to about 20 times greater than a width of at least one of the contraction portions.

In one form, at least one of the expansion portions has a width that is about 10 times to about 15 times greater than a width of at least one of the contraction portions.

In one form, at least one of the expansion portions of the gas flow path has a width of more than about 1.0mm.

In one form, at least one of the expansion portions of the gas flow path has a width of between about 3.0mm to about 4.0mm inclusive.

In one form, at least one of the expansion portions of the gas flow path has a width of about 3.5mm.

In one form, the inlet comprises a flow directing element that directs gas flow to at least one of the sound attenuating structures.

In one form, the inlet comprises a flow directing element that directs gas flow to at least one of the expansion chambers.

In one form, a constant distance is provided between the sound attenuating structures.

In one form, a variable distance is provided between the sound attenuating structures.

In one form, each sound attenuating structure has the same thickness.

In one form, at least one of the sound attenuating structures has a different thickness to one or more others of the sound attenuating structures.

In one form, each sound attenuating structure comprises a generally circular outwardly facing peripheral surface.

In one form, the diameter of each sound attenuating structure is generally equal to the diameter of the core.

In one form, the muffler further comprises a terminal end plate in which the outlet is located and wherein the outlet comprises one or more outlet apertures.

In one form, the distance from the inlet to the outlet corresponds to a sound frequency to be reduced, removed or dampened by the muffler.

In one form, the distance from the inlet to the outlet is at least about 20mm.

In one form, the distance from the inlet to the outlet is between about 20mm to about 100mm inclusive.

In one form, the muffler comprises one or more sound absorbing materials.

In one form, the muffler comprises an outlet end portion, on which the outlet is located, and wherein the outlet end portion comprises a sealing element adapted to seal against a surface of a respiratory device component.

In one form, the sealing element is located on an external surface of the muffler.

In one form, the muffler comprises an engagement mechanism to couple the muffler to a respiratory device component.

In one form, the respiratory device component comprises a pressure relief valve

In one form, the inlet comprises an engagement mechanism to engage a gas flow source.

In one form, the engagement mechanism comprises screw threads.

Also disclosed herein is a respiratory system muffler comprising: an inlet and an outlet; a shaft and at least one sound attenuating structure projecting from the shaft; and an internal wall located at a distance D1 from a portion of the at least one sound attenuating structure; wherein D1 > 0 to form a gap between the portion of the at least one sound attenuating structure and the internal wall.

In one form, the internal wall is located at a distance D2 from the shaft and wherein D2 > D1 to form an expansion chamber between the shaft and the internal wall.

In one form, the internal wall is an internal wall of a muffler housing.

In one form, the gap is about 0.5mm wide.

In one form, the gap has a width of between about 0.1mm to 0.5mm inclusive.

In one form, the gap is about 0.25 mm wide.

In one form, the inlet comprises one or more inlet apertures.

In one form, the sound attenuating structure projects generally laterally from the shaft.

In one form, the sound attenuating structure projects perpendicularly from the shaft.

In one form, the muffler comprises two or more sound attenuating structures and wherein an expansion chamber is defined between two adjacent ones of the two or more sound attenuating structures.

In one form, the gap between the sound attenuating structure and the internal wall comprises a contraction portion of the gas flow path and each expansion chamber comprises an expansion portion of the gas flow path to form a gas flow path comprising alternating contraction and expansion portions.

In one form, the expansion portion of the gas flow path comprises a first cross-sectional area and the contraction portion of the gas flow path comprises a second cross-sectional area and wherein the first cross-sectional area is at least two times larger than the second cross-sectional area of the gas flow path.

In one form, at least one of the expansion portions has a width that is about 10 times to about 20 times greater than a width of at least one of the contraction portions.

In one form, at least one of the expansion portions has a width that is about 10 times to about 15 times greater than a width of at least one of the contraction portions.

In one form, at least one of the expansion portions of the gas flow path has a width of more than about 1.0mm.

In one form, at least one of the expansion portions of the gas flow path has a width of between about 3.0mm to about 4.0mm inclusive.

In one form, at least one of the expansion portions of the gas flow path has a width of about 3.5mm.

In one form, the inlet comprises a flow directing element that directs gas flow to at least one of the sound attenuating structures.

In one form, the inlet comprises a flow directing element that directs gas flow to at least one of the expansion chambers.

In one form, a constant distance is provided between the sound attenuating structures.

In one form, a variable distance is provided between the sound attenuating structures.

In one form, each sound attenuating structure has the same thickness.

In one form, at least one of the sound attenuating structures has a different thickness to one or more others of the sound attenuating structures.

In one form, each sound attenuating structure comprises a generally circular outwardly facing peripheral surface.

In one form, the muffler further comprises a terminal end plate in which the outlet is located and wherein the outlet comprises one or more outlet apertures.

In one form, the distance from the inlet to the outlet corresponds to a sound frequency to be reduced, removed or dampened by the muffler.

In one form, the distance from the inlet to the outlet is at least about 20mm.

In one form, the distance from the inlet to the outlet is between about 20mm to about 100mm inclusive.

In one form, the muffler comprises one or more sound absorbing materials.

In one form, the muffler comprises an outlet end portion, on which the outlet is located, and wherein the outlet end portion comprises a sealing element adapted to seal against a surface of a respiratory device component.

In one form, the sealing element is located on an external surface of the muffler.

In one form, the muffler comprises an engagement mechanism to couple the muffler to a respiratory device component.

In one form, the respiratory component device comprises a pressure relief valve.

In one form, the inlet comprises an engagement mechanism to engage a gas flow source.

In one form, the engagement mechanism comprises screw threads.

Also disclosed herein is a respiratory system muffler comprising: an inlet, an outlet, and a core extending between the inlet and the outlet; wherein the core is spaced from an internal wall of the muffler to form a gap between a peripheral surface of the core and the internal wall; wherein the gap comprises a gas flow passage that forms a contraction portion of a gas flow path passing between the inlet and the outlet.

In one form, the gap is less than about 0.5mm wide.

In one form, the gap has a width between about 0.1mm to 0.5mm inclusive.

In one form, the gap is about 0.25mm wide.

In one form, the inlet comprises one or more inlet apertures.

In one form, the core comprises a shaft and wherein at least one sound attenuating structure projects generally laterally from the shaft.

In one form, the sound attenuating structure projects perpendicularly from the shaft.

In one form, the muffler comprises two or more sound attenuating structures.

In one form, an expansion chamber is defined between two adjacent ones of the two or more sound attenuating structures, and wherein the expansion chamber forms an expansion portion of the gas flow path passing between the inlet and the outlet to provide the gas flow path with alternating expansion and contraction portions.

In one form, the expansion portion of the gas flow path comprises a first cross-sectional area and the contraction portion of the gas flow path comprises a second cross-sectional area and wherein the first cross-sectional area is at least two times larger than the second cross-sectional area of the gas flow path.

In one form, at least one of the expansion portions has a width that is about 10 times to about 20 times greater than a width of at least one of the contraction portions.

In one form, at least one of the expansion portions has a width that is about 10 times to about 15 times greater than a width of at least one of the contraction portions.

In one form, at least one of the expansion portions of the gas flow path has a width of more than about 1.0mm.

In one form, at least one of the expansion portions of the gas flow path has a width of between about 3.0mm to about 4.0mm inclusive.

In one form, at least one of the expansion portions of the gas flow path has a width of about 3.5mm.

In one form, the inlet comprises a flow directing element that directs gas flow to at least one of the sound attenuating structures.

In one form, the inlet comprises a flow directing element that directs gas flow to at least one of the expansion chambers.

In one form, a constant distance is provided between the sound attenuating structures.

In one form, a variable distance is provided between the sound attenuating structures.

In one form, each sound attenuating structure has the same thickness.

In one form, at least one sound attenuating structure has a different thickness to one or more others of the sound attenuating structures.

In one form, the core is generally cylindrical.

In one form, each sound attenuating structure comprises a generally circular outwardly facing peripheral surface.

In one form, the diameter of each sound attenuating structure is generally equal to the diameter of the core.

In one form, the muffler further comprises a terminal end plate in which the outlet is located and wherein the outlet comprises one or more outlet apertures.

In one form, the distance from the inlet to the outlet corresponds to a sound frequency to be reduced, removed or dampened by the muffler.

In one form, the distance from the inlet to the outlet is at least about 20mm.

In one form, the distance from the inlet to the outlet is between about 20mm to about 100mm inclusive.

In one form, the muffler comprises one or more sound absorbing materials.

In one form, the muffler comprises an outlet end portion, on which the outlet is located, and wherein the outlet end portion comprises a sealing element adapted to seal against a respiratory device component.

In one form, the sealing element is located on an external surface of the muffler.

In one form, the muffler comprises an engagement mechanism to couple the muffler to a respiratory device component.

In one form, the respiratory device component comprises a pressure relief valve

In one form, the inlet comprises an engagement mechanism to engage a gas flow source.

In one form, the engagement mechanism comprises screw threads.

Also disclosed herein is a respiratory system muffler body to be received within a respiratory system muffler housing and to form a gas flow path with the muffler housing, the muffler body comprising: an inlet end portion to receive a gases flow; an outlet end portion to deliver a gases flow; one or more sound attenuating structures between the inlet and outlet portions; wherein one or more expansion portions and one or more contraction portions are formed along a portion of the gas flow path when the muffler body is received within the muffler housing.

In one form, the one or more sound attenuating structures extend laterally from a shaft connecting the inlet and outlet portions.

In one form, the shaft is a central shaft.

In one form, the one or more sound attenuating structures is substantially annular.

In one form, the muffler comprises two or more sound attenuating structures.

In one form, the two or more sound attenuating structures are spaced apart along a longitudinal axis of the insert.

In one form, the inlet end portion comprises one or more inlet apertures.

In one form, the inlet end portion comprises a sealing element configured to seal against a portion of the muffler housing.

In one form, the inlet end portion comprises a flow directing element.

In one form, the outlet end portion comprises one or more outlet apertures.

In one form, the outlet end portion comprises a terminal end plate.

In one form, the outlet comprises one or more outlet apertures arranged on the terminal end plate.

### BRIEF DESCRIPTION OF THE DRAWINGS

Embodiments will now be described by way of example only and with reference to the accompanying drawings in which:
Figure 1 is a schematic representation of one form of respiratory support system that may be suitable for use with a muffler of a pressure relief valve according to the invention;
Figure 2 is a perspective view of one form of muffler body of a muffler according to the disclosure;
Figure 3 is a side view of the muffler body of Figure 2;
Figure 4 is an end view showing the second end/outlet end of the muffler body of Figure 2;
Figure 5 is an illustrative cross-sectional side view of the muffler body of Figure 2 and showing one form of inlet that may be used with the muffler;
Figure 6a is a perspective view of one form of muffler comprising gas flow passages formed through the sound attenuating structures, extending from a first side surface to a second side surface of each sound attenuating structure.;
Figure 6b is a side view of one form of muffler comprising expansion chambers and sound attenuating structures of variable sizes;
Figure 6c is a perspective view of one form of muffler comprising gas flow passages formed in the outer peripheral surface of the sound attenuating structures;
Figure 7 is a perspective view of another form of muffler comprising a seal at the inlet end portion of the muffler and having different sized sound attenuating structures;
Figure 8 is a side view of the muffler of Figure 7;
Figure 9 is an illustrative cross-sectional side view of one form of pressure relief valve according to an exemplary embodiment of the invention, comprising an inlet that is coupled to a muffler according to the disclosure;
Figure 10 is an enlarged illustrative cross-sectional side view of one form of muffler located within an inlet of a respiratory system component;
Figure 11 is an illustrative cross-sectional side view of one form of muffler coupled to an inlet of another form of pressure relief valve according to an exemplary embodiment of the invention;
Figure 12 is an enlarged illustrative cross-sectional side view of showing one form of muffler comprising a muffler body and housing that are integrally formed as a single part;
Figure 13 is a perspective view of another form of muffler body according to the invention, the muffler body comprising a central chamber;
Figure 14 is a perspective view of another form of muffler having a central chamber, a series of expansion chambers of equal size and sound attenuating structures located within the periphery of the muffler core, and also having a bevelled surface at the inlet end portion of the muffler;
Figure 15 is a side view of the muffler of Figure 14;
Figure 16 is another side view of the muffler shown in Figure 14, showing a central shaft within the muffler core;
Figure 17 is a perspective view of another form of muffler, similar to that of Figure 14 but comprising smaller chamber apertures and also comprising sound attenuating structures;
Figure 18 is a side view of the muffler of Figure 17;
Figure 19 is a perspective view of another form of muffler comprising four central chambers of equal size;
Figure 20 is an end view, from the inlet end, of the muffler of Figure 19;
Figure 21 is a side view of a muffler, similar to that of Figure 19, but comprising larger chamber apertures;
Figure 22 is a side view of another form of muffler, comprising chamber apertures of different sizes;
Figure 23 is a perspective view of another form of muffler comprising a bevelled surface at the inlet end portion of the muffler core and comprising a central chamber;
Figure 24 is an end view, from the inlet end, of the muffler of Figure 23 showing outlet apertures located within the central chamber;
Figure 25 is a perspective view of another form of muffler comprising a bevelled surface at the inlet end portion of the muffler, four inlet apertures leading to a central chamber within a central shaft of the core and comprising sound attenuating structures of different sizes;
Figure 26 is a side view of the muffler shown in Figure 25;
Figure 27 is a perspective view of yet another form of muffler, which is similar to that of Figure 25, but comprises an inlet member at the inlet end portion of the muffler;
Figure 28 shows a schematic cross-sectional view taken transverse to the length of the muffler and through the central shaft and sound attenuating structure of the muffler and the muffler housing of Figure 11; and
Figure 29 shows a schematic cross-sectional view taken transverse to the length of the muffler and through the central shaft of the muffler and the muffler housing of Figure 11.

### DETAILED DESCRIPTION

The muffler forming part of the pressure relief valve of the invention is for use with a respiratory support system such as CPAP or high flow respiratory gas systems, for example a high flow system for use in anaesthesia procedures. Respiratory systems in which the muffler may be particularly useful are CPAP, BiPAP, high flow therapy, varying high flow therapy, low flow air, low flow O₂ delivery, bubble CPAP, apnoeic high flow (i.e. high flow to anesthetized patients), invasive ventilation and non-invasive ventilation. The muffler may also be used in surgical systems (that may comprise a carbon dioxide gases supply). Further, a muffler as described herein may be useful in systems other than respiratory systems. A muffler according to embodiments described herein is particularly adapted for use with a pressure relief or regulating device.

Unless the context suggests otherwise, a flow source provides a flow of gases at a set flow rate. A set flow rate may be a constant flow rate, variable flow rate or may be an oscillating flow rate, for example a sinusoidal flow rate or a flow rate with a step or square wave profile.

'High flow therapy' as used in this disclosure may refer to delivery of gases to a patient at a flow rate of greater than or equal to about 5 or 10 litres per minute (5 or 10 LPM or L/min).

Directional terminology used in the following description is for ease of description and reference only, it is not intended to be limiting. For example, the terms 'front', 'rear', 'upper', 'lower', 'top', 'bottom' and other related terms refer to the location of a part or portion of the article being described, when the article is in use.

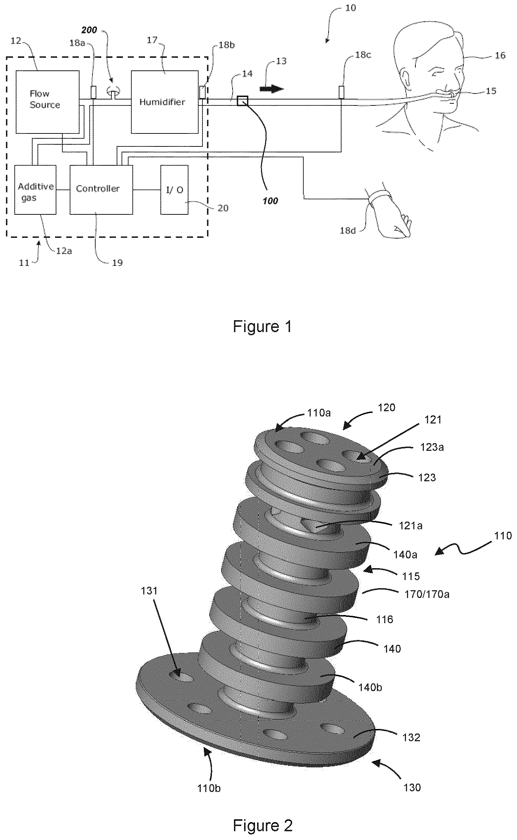

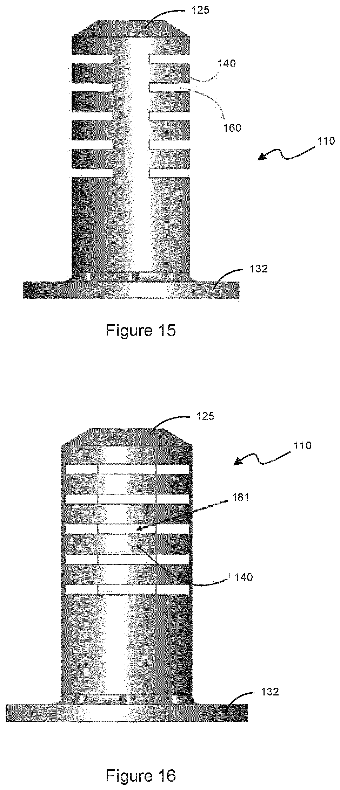

Various embodiments and methods of manufacture will now be described with reference to Figures 1 to 29. In these figures, like reference numbers are used to indicate like features.

Figure 1 shows one example of a respiratory system that may utilise the muffler of a pressure relief valve of the invention. The respiratory system/apparatus 10 comprises an integrated or separate component based arrangement, generally shown in the dotted box 11 in Figure 1. In some configurations, the system 10 could comprise a modular arrangement of components. Hereinafter the system/apparatus 10 will be referred to as system, but this should not be considered limiting. The system 10 may include a flow source 12, such as an in-wall source of oxygen, an oxygen tank, a blower, a flow therapy apparatus, or any other source of oxygen or other gas. The system 10 may also comprise an additive gas source 12a, comprising one or more other gases that can be combined with the flow source 12. The flow source 12 can provide a pressurised high gas flow 13 that can be delivered to a patient 16 via a delivery conduit 14, and patient interface 15 (such as a nasal cannula). A controller 19 controls the flow source 12 and additive gas source 12a through valves or the like to control flow and other characteristics such as any one or more of pressure, composition, concentration, volume of the high flow gas 13. A humidifier 17 is also optionally provided, which can humidify the gas under control of the controller and control the temperature of the gas. One or more sensors 18a, 18b, 18c, 18d, such as flow, oxygen, pressure, humidity, temperature or other sensors can be placed throughout the system and/or at, on or near the patient 16. The sensors can include a pulse oximeter 18d on the patient for determining the oxygen concentration in the blood.

The controller 19 may be coupled to the flow source 12, the additive gas source 12a, humidifier 17 and sensors 18a-18d. The controller 19 can operate the flow source to provide the delivered flow of gas. It can control the flow, pressure, composition (where more than one gas is being provided), volume and/or other parameters of gas provided by the flow source based on feedback from sensors. The controller 19 can also control any other suitable parameters of the flow source to meet oxygenation requirements. The controller 19 can also control the humidifier 17 based on feedback from the sensors 18a-18d. Using input from the sensors, the controller can determine oxygenation requirements and control parameters of the flow source 12 and/or humidifier 17 as required. An input/output (I/O) interface 20 (such as a display and/or input device) is provided. The input device is for receiving information from a user (e.g. clinician or patient) that can be used for determining oxygenation requirements. In some embodiments, the system may be without a controller and/or I/O interface. A medical professional such as a nurse or technician may provide the necessary control function.

The pressure may also be controlled. As noted above, the high gas flow (optionally humidified) can be delivered to the patient 16 via a delivery conduit 14 and the patient interface 15 or 'interface', such as a cannula, mask, nasal interface, oral device or combination thereof. In some embodiments, the high gas flow (optionally humidified) can be delivered to the patient 16 for surgical uses, e.g. surgical insufflation. In these embodiments, the 'interface' could be a surgical cannula, trocar, or other suitable interface. The patient interface can be substantially sealed, partially sealed or substantially unsealed. A nasal interface as used herein is a device such as a cannula, a nasal mask, nasal pillows, or other type of nasal device or combinations thereof. A nasal interface can also be used in combination with a mask or oral device (such as a tube inserted into the mouth) and/or a mask or oral device (such as a tube inserted into the mouth) that can be detached and/or attached to the nasal interface. A nasal cannula is a nasal interface that includes one or more prongs that are configured to be inserted into a patient's nasal passages. A nasal cannula may be a sealing nasal cannula or non-sealing nasal cannula. A mask refers to an interface that covers a patient's nasal passages and/or mouth and can also include devices in which portions of the mask that cover the patient's mouth are removable, or other patient interfaces such as laryngeal mask airway or endotracheal tube. A mask also refers to a nasal interface that includes nasal pillows that create a substantial seal with the patient's nostrils. The controller controls the system to provide the required oxygenation.

The system 10 may also include a pressure relief or regulating device, or pressure limiting device 200 (herein a pressure relief valve or PRV). The PRV may be placed anywhere in the system between the flow source 12 and the patient 16. In some forms, the PRV 200 is provided at an outlet of the flow source 12, or between the flow source 12 and the humidifier 17, for example near to an inlet of the humidifier 17. In some embodiments, the PRV 200 may be provided at an outlet of the humidifier 17 and/or an inlet to the conduit 14, or at any point along the conduit 14 through a suitable housing or coupling device. The PRV 100 may be located anywhere in the system, for example the PRV could be part of the patient interface 15. The system may additionally or alternatively include a flow controlled pressure relief or pressure regulating device (FCPRV). The PRV 200 may be a valve having features described in WO/2018/033863.

The system 10 may also include a muffler 100, according to the invention. The muffler 100 may be located anywhere along the gas flow path of the system, between the flow source 12 and the patient 16. In some forms, the muffler 100 is provided at the outlet of the flow source 12, the inlet or outlet of the humidifier 17, the inlet or outlet of the pressure relief valve 200, or within the gas delivery conduit 14 at any location upstream or downstream from the humidifier or pressure relief valve. In one form, the muffler may be provided at the outlet of the flow source 12. In the embodiment illustrated in Figure 1, the muffler 100 is provided along the gas delivery conduit downstream of the humidifier 17.

Figures 2 to 29 show embodiments of respiratory mufflers that may be used to dampen noise within a respiratory support system.

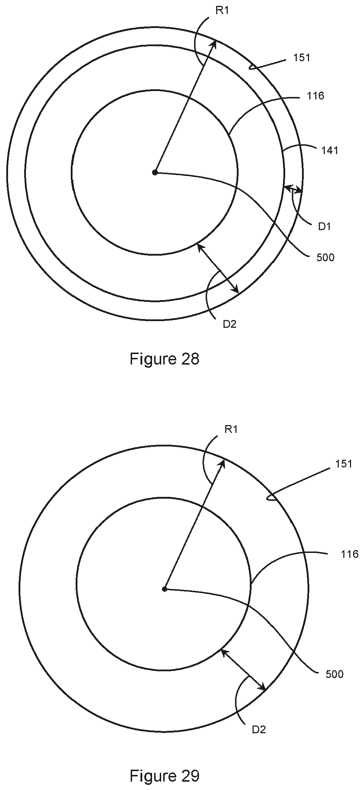

As shown best in Figures 2 to 29, the muffler 100 comprises a body 110 having an inlet 120, an outlet 130, and at least one sound attenuating structure 140 located between the inlet 120 and the outlet 130. The body 110 comprises a longitudinal central axis 500 that extends centrally along the length of the body 110 between the inlet 110 and the outlet 120, as shown in Figures 28 and 29. The muffler 100 also comprises a housing 150 within which at least a portion of the muffler body 110 is inserted or otherwise located.

The muffler defines a gas flow path that extends between the inlet 120 and the outlet 130. The gas flow path comprises a variable lateral cross-sectional area along its length to provide contraction and expansion portions to attenuate noise. The variable lateral cross-sectional areas may provide the gas flow path with variable volumes along its length. Alternatively or additionally, the gas flow path may define a tortuous route between the inlet 120 and the outlet 130 to attenuate noise.

The term 'lateral cross-sectional area' as used in this disclosure may refer to an area of the gas flow path that is generally transverse to and generally perpendicular to the general direction of gas flow at that portion of the gas flow path in which the lateral cross-sectional area is located. For example, the lateral cross-sectional area of the gas flow path may be transverse to the longitudinal axis of the muffler body.

In some forms, the muffler body 110 comprises a core 115 comprising a first end (an inlet end) 110a, and a second end (an outlet end) 110b. The central longitudinal axis of the muffler body may extend centrally through the length of the core.

The muffler body 110 comprises an inlet end portion, at which the inlet 120 is located, and an outlet end portion, at which the outlet 130 is located. Typically, the inlet 120 and outlet 130 are located at opposite ends of the muffler body.

The inlet 120 may comprise one or more inlet apertures 121 through which gas may enter the gas flow path of the muffler 100. In one form, as shown in Figure 2, the muffler comprises four inlet apertures 121.

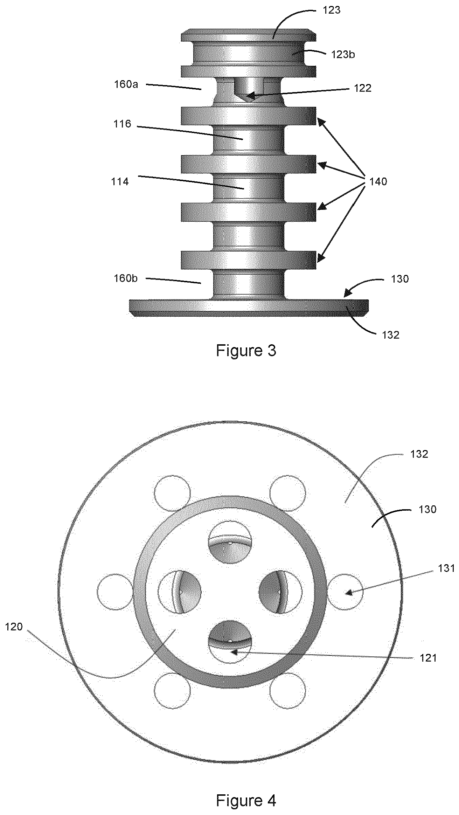

The outlet 130 may comprise one or more outlet apertures 131. In one form, as shown in Figure 4, the muffler comprises six outlet apertures 131.

In some forms, the muffler body 110 comprises an elongate core 115 that extends between the inlet 120 and the outlet 130. The core may comprise a central shaft 114 comprising an outwardly facing side surface 116 extending along the sides of the shaft between the first and second ends 110a, 110b of the muffler body. In some forms, the shaft 114 and core 115 each comprise a cylindrical shape. For example, the core 115 may comprise a central cylindrical shaft. The core 115 is configured to be at least partially or fully received within the muffler housing 150. In some forms, the core 115 is configured to be located generally concentrically within the muffler housing 150.

In some forms, the core 115 comprises one or more sound attenuating structures 140 that are located between the inlet 120 and the outlet 130. In one form, as shown in Figures 2 to 8, the muffler core comprises one or more projections that form sound attenuating structures 140 that project outwardly from the side surface 116 of the shaft 114. The sound attenuating structures 140 may each comprise a peripheral surface 141, which may form a peripheral edge of the sound attenuating structure.

In one form, a terminal end plate 132 is provided at the second end (the outlet end) of the muffler body 110. The outlet 130 is located at the terminal end plate. The terminal end plate 132 may or may not be configured to seal against a surface of the muffler housing 150, such as an internal wall 151 of the housing, and/or against a surface of another respiratory device component.

In some forms, the outlet 130 may comprise one or more outlet apertures 131 that are located in the terminal end plate 132. In one form, multiple outlet apertures 131 are provided in a terminal end plate 132. In one form, the terminal end plate 132 may comprise a circular peripheral surface/edge that is sized and shaped to seal against a portion of the muffler housing 150. In this form, gas flow is forced to exit the muffler through the outlet apertures 131.

Alternatively or additionally, the outlet 130 may comprise at least one outlet aperture that comprises an outlet gap between a peripheral surface 132a of the terminal end plate 132 and the muffler housing 150 so that gas can exit the muffler via the outlet gap. In this form, the terminal end plate does not seal with the muffler housing and a small amount of gas may leak between the terminal end plate and the muffler housing 150. The peripheral surface 132a may be a peripheral edge of the terminal end plate 132.

The muffler housing 150, as referred to in this specification, may be a dedicated muffler housing or it may be a tubular conduit (such as a gas delivery tube), or an inlet or an outlet of another component of a respiratory system. For example, the muffler housing may be provided by an inlet 210 or outlet 220 of a pressure relief valve 200 or the inlet or outlet of a humidifier, or the outlet of a gas source.

Typically, the muffler housing 150 comprises a hollow interior region to receive at least a portion of the muffler body, such as the core 115. The hollow interior is at least in part defined by an internal wall 151 of the housing 150. Typically, the muffler housing 150 comprises a cylindrical internal wall 151 to form a cylindrical hollow region in which the muffler core 115 may be located. Where the muffler 100 comprises a cylindrical core 115, the core may be concentrically located within the muffler housing 150 so that the core 115 and housing 150 are located along the same central longitudinal axis. The internal wall 151 may generally face toward the central longitudinal axis of the muffler.

In some forms, the terminal end plate 132 extends across the width/diameter of the muffler body 110 so as to be equal to or greater than the width/diameter of the sound attenuating structure(s) 140. In other forms, the width/diameter of the terminal end plate 132 may be less than the width/diameter of the sound attenuating structure(s) 140.

In one form, the muffler comprises two or more sound attenuating structures 140. An expansion chamber 160 is provided between adjacent sound attenuating structures 140. Each expansion chamber 160 has walls defined by side surfaces of the two adjacent sound attenuating structures 140, a portion of the outer surface 116 of the muffler shaft 114 that lies between the adjacent sound attenuating structures, and an internal wall 151 of the muffler housing 150. Each expansion chamber 160 forms a portion of the gas flow path through the muffler. Each expansion chamber 160 forms an expansion portion of the gas flow path and comprises a first lateral cross-sectional area through which gas flow passes. Where the muffler comprises multiple expansion chambers 160, the gas flow path will have multiple expansion portions. Gas enters each expansion chamber 160 from a portion of the gas flow path that comprises a second lateral cross-sectional area that is smaller than the first lateral cross-sectional area of the expansion chamber 160. Therefore, the gas is caused to expand when it reaches an expansion chamber 160.

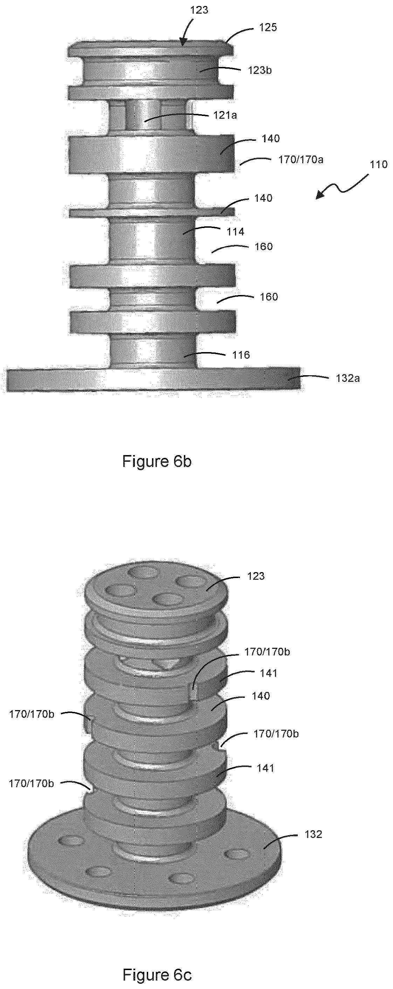

In some forms, the sound attenuating structures 140 are spaced equidistant apart to provide a constant expansion chamber volume along the length of the muffler, as shown in Figures 1 to 5, and 14 to 22. In other forms, a variable distance is provided between the sound attenuating structures to provide variable expansion chamber volumes along the length of the muffler, as shown in Figure 6b.

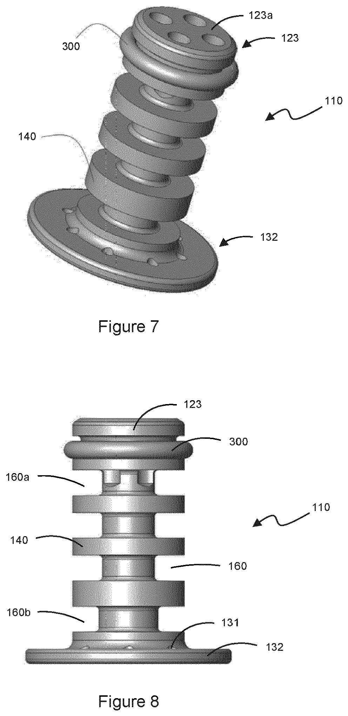

The sound attenuating structures 140 may have the same thickness. Alternatively, each sound attenuating structure 140 may have a different thickness to one or more other sound attenuating structures of the muffler. In some forms, the thickness of the sound attenuating structures 140 may increase toward one end of the muffler. For example, embodiments illustrated in Figures 7, 8 and 25 to 27, show a muffler comprising a series of sound attenuating structures 140 that increase in thickness toward the outlet end of the muffler. Figure 6b also shows a muffler comprising sound attenuating structures of different thicknesses.

In some forms, as shown in Figures 1 to 8, one or more of the sound attenuating structures 140 project generally laterally from the outwardly facing side surface 116 of the shaft 114. For example, the sound attenuating structure(s) may project at an angle, such as substantially perpendicularly (with respect to the longitudinal axis extending through the core) from the shaft 114.

In some forms, the sound attenuating structure(s) 140 project(s) toward the internal wall 151 of the muffler housing 150. In some forms, a portion of the sound attenuating structure(s) 140 may contact the internal wall 151. In some forms, a portion of the sound attenuating structure may be configured to seal against the internal wall 151. For example, a sealing member, such as an o-ring or another form of annular seal may be located around the outwardly facing peripheral surface 141 of a disc shaped sound attenuating structure 140 to seal against the internal wall 151 of the muffler housing 150.

In some forms, one or more sound attenuating structures may project from an internal wall of the muffler housing toward the body of the muffler. In other forms, one or more sound attenuating structures may project from the internal wall toward the muffler body and one or more other sound attenuating structures may form part of the muffler body, such as by projecting from a core of the muffler body.

Each sound attenuating structure 140 may define a gas flow passage opening 170 that defines a gas flow passage around and/or through a sound attenuating structure 140 of the muffler. The gas flow passage forms a portion of the gas flow path through the muffler 100.

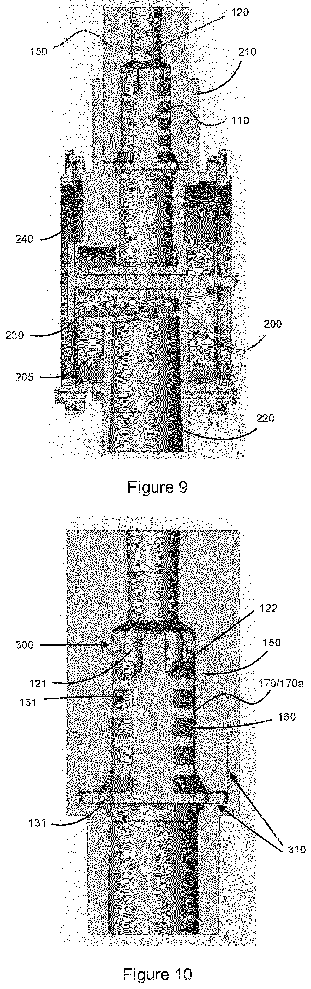

In some forms, the sound attenuating structure(s) 140 terminate(s) proximate to the internal wall 151 of the muffler housing 150 to form a gas flow passage opening 170 defined by a gap between the peripheral surface 141 of each sound attenuating structure 140 and the internal wall 151. In some forms, as shown in Figures 2 to 27, the sound attenuating structures 140 each comprise a peripheral surface/edge 141 that is shaped and sized to provide a gap between the sound attenuating structure 140 and an internal wall 151 of the muffler housing 150. For example, the sound attenuating structures 140 may be generally disc shaped, having a circular or annular outer peripheral surface 141 with a diameter that is smaller than the diameter of the hollow interior region of the muffler housing 150, to provide a gap 170a between the structure 140 and the internal wall 151 of the housing 150, as shown in Figures 9 through 12. The gap forms a gas flow passage opening 170 through which gas may flow.

Each gas flow passage opening 170 defines a contraction portion of the gas flow path. The gas flow passage openings 170 formed by multiple sound attenuating structures 140 may be the same size and/or shape or the openings 170 may be different sizes and/or shapes.

In some forms, the width of the gap 170a between the peripheral surface 141 of a sound attenuating structure 140 and the internal wall 151 of the housing 150 may be about 0.5 mm or less. For example, the width may be between about 0.1 mm to about 0.5 mm inclusive. In one form, the width of the gap may be about 0.25 mm. In some forms of muffler, the widths/diameters of the sound attenuating structures 140 may vary and/or the internal width/diameter of the internal wall 151 or part thereof of the muffler housing 150 may vary so that the width of the gap may vary between the peripheral surfaces 140a of different sound attenuating structures 140 and the internal wall 151 of the muffler housing 150.

In some forms, the sound attenuating structure(s) 140 comprise(s) a solid front surface that faces the inlet end of the muffler and that substantially blocks gas flow through the muffler 100. In this form, the sound attenuating structure(s) 140 may terminate(s) proximate to the internal wall 151 of the muffler housing to form a gas flow passage opening 170, defined by a gap 170a between the peripheral surface 141 of the sound attenuating structure(s) 140 and the internal wall 151, as described above. Alternatively or additionally, at least a portion of the peripheral surface 141 of the sound attenuating structure(s) may contact the internal wall 151 of the housing 150 and at least a portion of the peripheral surface 141 may comprise one or more recesses, channels or troughs to define one or more gas flow passage openings 170b that allow gas to flow around the sound attenuating structures 140 from the muffler inlet 120 to the outlet 130. In these embodiments, the gas flow path opening(s) 170 provide(s) the only route for gas to flow around the sound attenuating structure(s).

In another form, the peripheral surface 141 of the sound attenuating structures 140 may be sized and shaped so that the lateral cross-sectional area of any opening/gap formed between the peripheral surface 141 and an internal wall 151 of the muffler housing varies in size around the peripheral surface 141. For example, the sound attenuating structures 140 may comprise an undulating or varying peripheral surface 141 that comprises a series of recesses, such as troughs or channels, as shown in Figure 6c. Each recess may define a gas flow passage opening 170b, which comprises a portion of the gas flow path through the muffler. For example, the sound attenuating structures 140 of the muffler may be generally clover shaped with recesses forming gas flow passage openings 170b between the 'leaves' of the clover.

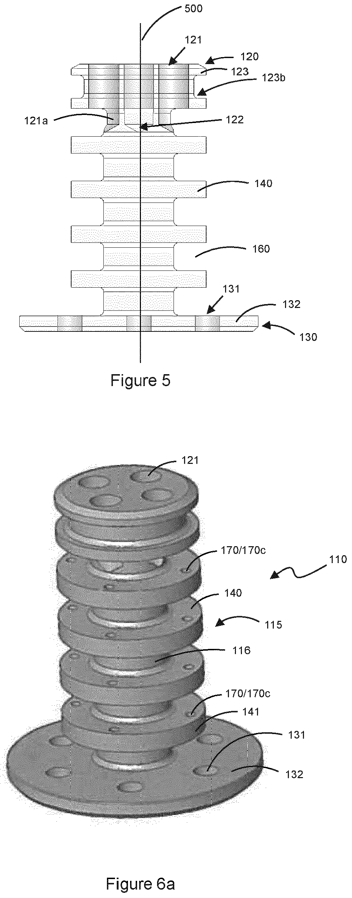

In yet another form, as shown in Figure 6a, the muffler may be configured so that a gas flow passage may pass through gas flow passage openings 170c formed in the sound attenuating structure(s) 140. Each sound attenuating structure 140 may comprise at least one gas flow passage opening 170c, such as an aperture, gap, hole channel, or slot, for gas to flow through the sound attenuating structure. In this form, gas may flow through a sound attenuating structure 140 from a first side (an inlet side) of the structure 140 to a second side (an outlet side) of the structure.

In one form, the muffler may comprise one or more gas flow passage openings 170 defined by a gap 170a between a peripheral surface 141 of at least one sound attenuating structure 140 of the muffler and the internal wall 151 of the muffler housing 150 and/or one or more gas flow passage openings 170b, each comprising a recess, channel, trough or the like, may be formed in the outwardly facing peripheral surface 141 of one or more sound attenuating structures 140 of the muffler and/or the muffler may comprise one or more gas flow passage openings 170c formed through the sound attenuating structure(s) 140.

In some forms, one or more sound attenuating structures 140 may comprise multiple gas flow passage openings 170. The gas flow passage openings 170 formed within a single sound attenuating structure 140 and/or the gas flow passage openings 170 provided by two or more adjacent sound attenuating structures 140 may be of the same or different sizes and shapes. Gas flow passage openings 170 of one sound attenuating structure 140 may be aligned with or offset from gas flow passage openings 170 of an adjacent sound attenuating structure 140.

One or more of the gas flow passage opening(s) 170 may be offset from the inlet opening(s) 121 and/or the outlet aperture(s) 131 of the muffler to provide a tortuous gas flow path. For example, in some forms, where the gas flow passage opening(s) 170 in the sound attenuating structure(s) 140 align with the inlet aperture(s) 121 and the outlet aperture(s) 131, the gas may follow a direct flow path through the muffler 100, expanding and contracting along the length of the flow path. However, in forms where the gas flow passage opening(s) 170 in the sound attenuating structure(s) 140 is/are offset from the inlet aperture(s) 121 and/or the outlet aperture(s) 131 then the gas flow path defines a tortuous route between the inlet 120 and the outlet 130, as the gas also expands and contracts along the length of the gas flow path and potentially bounces around off internal surfaces of the muffler 100. In this arrangement, the gas flow passage opening(s) 170 each define a first portion/contraction portion of the tortuous gas flow path and the expansion chambers 160 each define a second portion/expansion portion of the gas flow path. The tortuous flow path may help attenuate sound. Additionally or alternatively, one or more of the gas flow passage opening(s) 170 in a sound attenuating structure 140 may be offset from one or more gas flow passage opening(s) 170 in another sound attenuating structure 140.

Each gas flow passage opening 170 may form a contraction portion of the gas flow path. The contraction portion may pass between two expansion portions defined by expansion chambers and may comprise a second lateral cross-sectional area through which gas flow passes. The second lateral cross-sectional area of the gas flow path is less than the first lateral cross-sectional area of the flow path at each expansion portion. In this arrangement, gas flow passing through a gas passage opening 170 is caused to contract and therefore increase in pressure. The gas flow then expands and decreases in pressure when the gas flow enters the following expansion chamber 160. Therefore, gas flowing along the gas flow path is caused to alternately contract and expand as the gas flows through the gas flow passage openings 170 and the expansion chambers 160 located between the inlet 120 and outlet 130 of the muffler 100. In some forms, the width of the gas flow passage opening 170 is between more than 0mm to about 0.5mm (inclusive) and is optionally about 0.25mm wide.

Where the muffler comprises multiple gas flow passage openings 170, the openings 170 may each comprise the same lateral cross-sectional area or different lateral cross-sectional areas. Similarly, where the muffler 100 comprises multiple expansion chambers 160, the expansion chambers 160 may each comprise the same lateral cross-sectional areas or different lateral cross-sectional areas. As the gas moves between a contracted state and an expanded state and vice versa, sound produced from gas flow is attenuated. Therefore, it may be beneficial to some respiratory systems to provide mufflers 100 that define a gas flow path that repeatedly causes the gas to (optionally alternately) contract and expand between the muffler inlet 120 and outlet 130, such as by providing a series of alternating sound attenuating structures 140 and expansion chambers 160.

In some forms, the first lateral cross-sectional area is at least two times larger than the second lateral cross-sectional area of the gas flow path. In other words, the lateral cross-sectional area of the expansion chamber 160 or expansion portion may be at least two times larger than the lateral cross-sectional area of the gas flow passage 170 or contraction portion passing through a sound attenuating structure 140 or passing between the peripheral surface of a sound attenuating structure 140 and an internal wall 151 of the muffler housing 150.

In terms of ratios of the lateral cross-sectional areas, the ratio between the first lateral cross-sectional area of the expansion portion and the second lateral cross-sectional area of the contraction portion is more than about 2. In one embodiment, the ratio between the first lateral cross-sectional area of the expansion chamber 160 and the second lateral cross-sectional area of a gas flow passage opening 170 being 0.25mm wide is about 10. The ratio between the lateral cross-sectional area of the expansion chamber 160 and that of the gas flow passage opening 170 may comprise a value greater than 2. (based on the peripheral surface of a sound attenuating structure 140 having a maximum clearance of 0.5mm with the internal wall 151 of the muffler housing). In terms of the ratios of volume, in one embodiment, the ratio between the expansion chamber volume and the gas passage opening volume is about 20. Again, the volume ratio may be greater than 2.

In some forms, at least one of the expansion portions has a width that is about 10 times to about 20 times greater than a width of at least one of the contraction portions. Optionally, at least one of the expansion portions has a width that is about 10 times to about 15 times greater than a width of at least one of the contraction portions. In some forms, at least one of the expansion portions of the gas flow path has a width of more than about 1.0mm. Optionally, at least one of the expansion portions of the gas flow path has a width of between about 3.0mm to about 4.0mm inclusive. In some forms, at least one of the expansion portions of the gas flow path has a width of about 3.5mm.

Tests have shown that sound attenuation is improved in mufflers 100 having greater numbers of sound attenuating structures 140. However, each sound attenuating structure 140 also impacts on the pressure drop or the driving pressure across the muffler 100. Therefore a balance needs to be struck between the number of sound attenuating structures 140 used in a muffler and the maximum gas flow rates to be provided through the respiratory system, particularly flow rates to be provided to a respiratory system component, for example a pressure relief valve. Based on these tests, mufflers 100 comprising four sound attenuating structures 140 are preferred, but mufflers having fewer or greater numbers of sound attenuating structures 140 may be useful in different respiratory support systems.

In some forms, the muffler 100 comprises one or more flow directing elements 122 that direct gas flow in a desired direction. For example, one or more flow directing elements 122 may direct gas to a sound attenuating structure 140 along the gas flow path. Alternatively or additionally, one or more flow directing elements 122 may direct gas flow to an expansion chamber 160 or to a sound attenuating structure 140.

In one form, as shown in Figures 3 and 5, each inlet 120 may be configured to provide a flow directing element 122 that directs gas to the first sound attenuating structure 140a (located closest to the inlet) and/or to an expansion chamber 160 located between the inlet end of the muffler 100 and the first sound attenuating structure 140a.

In some forms, as shown in Figures 3 and 5, the muffler body 110 may comprise an inlet end portion comprising an inlet member 123. The inlet member 123 comprises an inlet end face 123a located at the first end/inlet end 110a of the muffler body 110. One or more inlet apertures 121 may be formed in the inlet end 110a.

In some forms, the muffler body 110 may comprise an elongate cylindrical core 115 having a central shaft 114 that extends between the inlet member and a terminal end plate 132 located at the outlet 130, as shown in Figures 2 to 27. The inlet apertures 121 may extend through the end face 123a of the inlet member 123 and may comprise exit openings 121a located in the side surface 116 of the muffler shaft 114 and between the inlet member 123 and the first sound attenuating structure 140.

An expansion chamber, referred to herein as an inlet expansion chamber 160a, may be provided between the inlet 120, such as between the inlet member 123, and the adjacent/first sound attenuating structure 140.

The inlet 120 may be configured to comprise a flow directing element 122 to direct gas flow to a first sound attenuating structure 140 (located closest to the inlet) and/or to the inlet expansion chamber 160a. For example, the exit openings 121a of the inlet apertures 121 may be directed toward a first surface of the first sound attenuating structure 140 or toward the inlet expansion chamber 160a. In other forms, the flow directing elements may direct gas flow toward one or more gas flow passages 170 provided by the first sound attenuating structure 140a. In some forms, where the muffler 100 comprises multiple inlet apertures 121, one or more of the inlet apertures 121 may comprise flow directing elements 122 that direct gas flow to a first surface of the first sound attenuating structure 140a or to the gas flow passage(s) 170 provided by the first sound attenuating structure 140a, and one or more others of the inlet apertures 121 may comprise flow directing elements 122 that direct gas flow to the inlet expansion chamber 160a.

The flow directing elements 122 may be of any suitable configuration and shape. In one form, as shown in Figures 2, 3 and 5, the flow directing elements 122 are formed by wall surfaces of the inlet apertures 121. For example, an inlet aperture 121 may be configured to taper or narrow toward its exit opening 121a. In one form, an inlet aperture 121 narrows to a point at its exit opening 121a so that the walls of the inlet aperture 121 form a triangular or conical shape at the exit opening 121a. In some forms, the walls of the inlet aperture 121 may be angled more than about 45°, for example between about 45° and about 70°, to help direct gas flow outwardly from the muffler core 115 and toward the side wall of the adjacent first sound attenuating structure. In yet another form, one or more deflectors may form flow directing elements 122 and may be located within one of more of the inlet apertures 121 or may be located near the exit openings 121a of the inlet apertures to direct gas flow through the muffler inlet 120.

The flow directing elements 122 may be configured to encourage gas flow to take a tortuous flow path from the muffler inlet 120 to the muffler outlet 130 and/or to cause the gas flow to contact surfaces of the muffler, such as by causing the gas flow to bounce or reflect off a side wall of a sound attenuating structure 140.

In some forms, the muffler housing 150 comprises an inlet portion having an internal wall surface defining a portion of the gas flow path through the muffler 100. In one form, the muffler body 110 within the muffler housing 150 may comprise one or more inlet apertures 121 that are offset from the gas flow path through the inlet portion of the muffler housing 150. In this form, the arrangement between the muffler housing inlet and the inlet apertures 121 of the muffler body 110 forms a tortuous gas flow path. The portion of the gas flow path through the muffler housing inlet may also have a lateral cross-sectional area that is larger than that of the gas flow path passing through each inlet aperture 121. In this arrangement, the muffler housing inlet may form an expansion portion of the gas flow path and the inlet apertures may each form a contraction portion of the gas flow path.

In some forms, an outlet expansion chamber 160b may be provided between the last sound attenuating structure 140b and the outlet 130. The outlet expansion chamber 160b comprises a portion of the gas flow path through the muffler and is the last expansion chamber along the gas flow path before gas is caused to exit the muffler through the outlet 130.

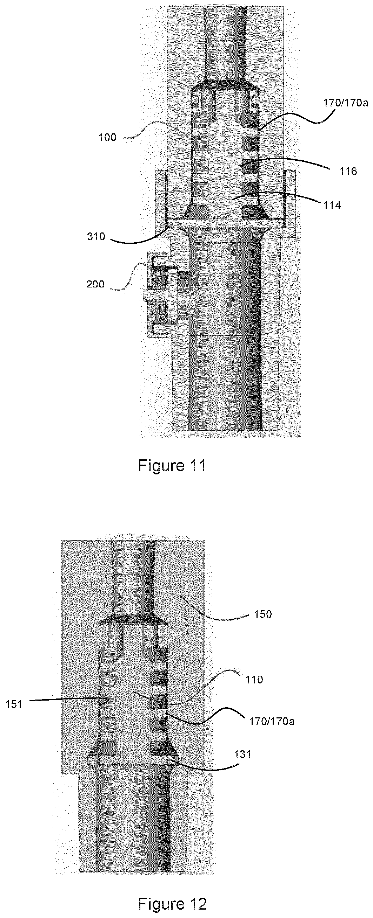

In some forms, the muffler body 110 comprises an insert that is configured to be at least partially received within a muffler housing 150, as shown in Figures 9 to 12. In one form, the muffler body forms an insert that is fitted within a housing 150 to form a muffler assembly, as shown in Figure 10. In other forms, the muffler body 110 and housing 150 may be integrally formed as a single part, as shown in Figure 12. For example, the muffler body 110 and housing 150 may be moulded together so as to be inseparable.

In some forms, the inlet end portion of the muffler body 110 comprises a first sealing element 300 that seals against an internal wall 151 of the housing 150. In some forms, the sealing element 300 comprises a flexible member, such as a rubber or elastic seal. In some forms, the sealing element 300 comprises an annular seal, such as an o-ring, an interference seal, adhesive, or any other suitable form of sealing that extends around at least a portion of the peripheral surface of the inlet end portion.

Where the muffler body 110 comprises an inlet end portion comprising an inlet member 123, the inlet member 123 may comprise a seal support 123b on which a seal may be located to seal the inlet end portion of the muffler 100 to an internal wall 151 of a muffler housing 150. In one form, the inlet member 123 comprises a cylindrical boss having a circular peripheral surface on which a channel is formed. The channel forms a seal support 123b that is configured to receive a sealing element 300, such as an o-ring seal, as shown in Figures 7 to 10 for example.

By sealing the inlet end portion of the muffler body 110 against the muffler housing 150, gas is forced to enter the muffler through the inlet apertures 121 and is then directed along the gas flow path through the muffler 100. However, it is not essential to seal the muffler inlet end portion with the housing 150. The muffler 100 may function with at least some degree of leaking between the muffler body 110 and the muffler housing 150, but sound attenuation performance is improved when seals are used. In some forms, a seal may not be used at the muffler inlet end portion, but in such embodiments, the muffler inlet end portion could be configured to minimise any gas leaks between the muffler inlet end portion and the housing 150. For example, the muffler inlet end portion may be sized and shaped to provide a snug fit with the internal wall 151 of the muffler housing 150. The size of the inlet aperture 121 may be adjusted to compensate for the flow around the muffler, such as if a seal is not used to form a seal between the inlet end portion and the valve housing.

In some forms, the outlet end portion of the muffler comprises a second sealing element 310 configured to seal against a surface of the muffler housing 150 and/or a surface of another respiratory device component. Typically, the sealing element 310 is located on an external surface of the muffler and comprises a flexible seal, such as an annular seal, which may comprise an o-ring, an interference seal, adhesive, or any other suitable form of sealing. In some forms, a terminal end plate 132 is provided at the outlet portion of the muffler and comprises a sealing element 310 configured to seal against a surface of the muffler housing 150.

In some forms, the muffler 100 comprises a seal at both the inlet end and the outlet end of the muffler. For example, a first sealing element 300 comprising an o-ring seal may be provided at the inlet end portion and a second sealing element 310 comprising an interference seal may be provided at the outlet end portion of the muffler. The seals may be configured to help retain the muffler body 110 generally centrally within the muffler housing 150 so that the core 115 of the muffler body is generally concentrically aligned with the internal wall 151 of the muffler housing 150. By maintaining the muffler core 115 and body 110 in a concentric position within the housing 150, gas flow may move evenly through the muffler. The seals may also direct gas flow through the inlet apertures 121 and the outlet apertures 131 to allow the muffler to achieve its desired or optimal performance. In some forms, the muffler inlet 120 may seal against the muffler housing 150 and the muffler outlet 130 may seal against the surface of a respiratory system component, such as an inlet port of a pressure relief valve.

An example of just one form of gas flow path passing through just one form of muffler of the disclosure will now be described. In this form, the muffler 100 comprises two or more sound attenuating structures 140 that include a first attenuating structure 140a located closest to the inlet 120 and a last sound attenuating structure 140b located closest to the outlet 130. Optionally, one or more other sound attenuating structures may be located between the first and last structures 140a, 140b. An expansion chamber 160 is provided between adjacent sound attenuating structures 140. Optionally, a first expansion chamber 160a is provided between the muffler inlet 120 and the first sound attenuating structure 140a. Optionally, an expansion chamber 160b is provided between the last sound attenuating structure 140b and the muffler outlet 130.

In this arrangement, gas may flow through the muffler inlet 120 and into the first expansion chamber 160a, where the gas is able to expand. The gas then flows through the gas flow passage(s) 170 defined by the first sound attenuating structure 140a. The gas pressure increases as the gas flows through the constricted portion of the flow path defined by the gas flow passage(s) 170, which may comprise a gap between the peripheral surface 141 of the first sound attenuating structure 140a and an internal wall 151 of the muffler housing 150. The gas then enters a second expansion chamber 160, where the gas is able to expand due to the larger lateral cross-sectional area of the expansion chamber 160 compared to the smaller lateral cross-sectional area of the gas flow passage(s) 170. Continual gas flow into the muffler inlet 120 forces the gas out of the second expansion chamber 160 and through the gas flow passage(s) 170 defined by the second sound attenuating structure 140. The gas flow passage(s) 170 of the second sound attenuating structure 140 may also comprise a gap between the peripheral surface 141 of the second sound attenuating structure 140 and the internal wall 151 of the housing 150. The process of expansion and contraction of the gas flow path continues until the gas flow passes through the gas flow passage(s) 170 of the last sound attenuating structure 140b and exits the muffler through the outlet 130.

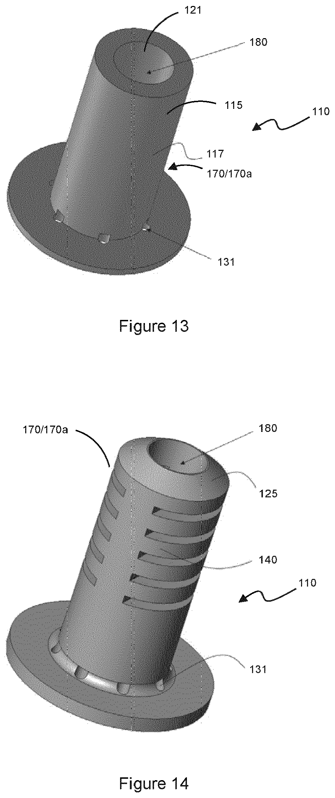

Embodiments shown in Figures 13 to 27 show alternative forms of muffler 100. As described above, the muffler comprises a muffler body 110, an inlet 120, an outlet 130, and a housing 150. At least one sound attenuating structure 140 may be located between the inlet 120 and the outlet 130,

In some forms, the muffler body 110 comprises a central elongate core 115, which may or may not be cylindrical and extends along a central longitudinal axis of the muffler 100, as described in relation to the embodiments disclosed above. The core 115 comprises an outer peripheral side surface 116 that extends along the length of the core.

The core 115 comprises an inlet end portion located at a first end/inlet end of the muffler body and in which the inlet 120 is located. The core also comprises an outlet end portion located at a second end/outlet end of the muffler body and in which the outlet 130 is located. A gas flow path is provided between the inlet 120 and outlet 130.

At least a portion of the gas flow path comprises a gas flow passage opening 170 that comprises a gap 170a defined by the side surface 117 of the core 115 (and in some forms also the outer peripheral edge/surface 141 of the sound attenuating structure(s)) and an internal wall 151 of the muffler housing 150 when the core 115 is inserted/located within the housing 150. The gas flow passage opening 170 allows gas to flow through the muffler from the inlet 120 to the outlet 130.

In some forms, the inlet 120 of the muffler may comprise a bevelled/chamfered surface 125 that angles toward the side surface 117 of the core 115, as shown in Figures 13 to 26. The bevelled surface 125 forms a flow directing element that directs gas flow towards one or more gas flow passage openings 170 of the muffler.

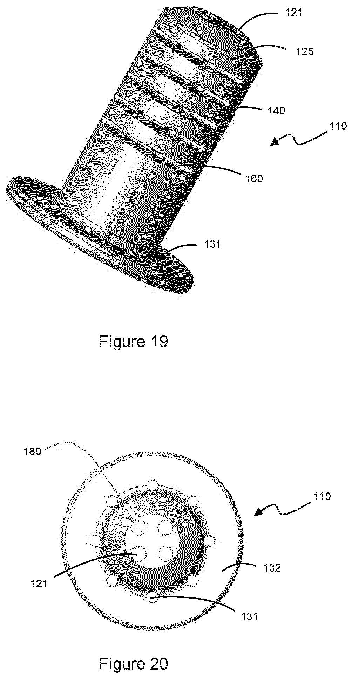

The inlet end of the core 115 comprises one or more inlet apertures 121. Each inlet apertures may lead to a central chamber 180 within the core 115. For example, the core may comprise multiple inlet apertures 121 and multiple central chambers 180, as shown in Figures 19 to 22, 25, and 27. Each central chamber 180 may extend along a portion of the length of the core 115 or along almost the whole of the length of the core 115. In some forms, each central chamber 180 terminates within the core 115 to form a blind end, which may be located at or near the outlet end of the muffler. In some forms, as shown in Figure 13 to 18, the core 115 is a generally tubular shape and comprises a single central chamber 180 that comprises a blind end at or near the outlet end of the muffler. For example, the muffler body may comprise a terminal end plate 132 that extends across the outlet end of the muffler to provide a blind end wall of the central chamber 180 of the core. Where the muffler comprises multiple central chambers 180, the terminal end plate 132 may extend across the outlet end of the muffler to provide a blind end wall to each central chamber 180.

In one form, the muffler body 110 may comprise a terminal end plate 132 comprising one or more outlet apertures 131, as shown in Figures 13 to 27. Typically, as shown in Figures 13 to 22 and 25 to 27, the outlet apertures 131 provided on the terminal end plate 132 are in fluid communication with the gas flow passage opening 170 when the muffler body 110 is located within the muffler housing 150. For example, Figure 20 shows one form of muffler comprising a terminal end plate 132, in which eight outlet apertures 131 are spaced equidistant around the core 115 to be in fluid communication with the gas flow passage 170 when the muffler is located within the muffler housing.

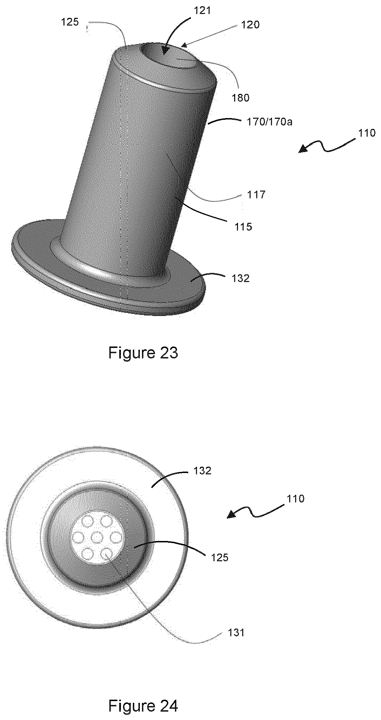

Alternatively or additionally, the outlet apertures 131 are provided on the terminal end plate 132 to be in fluid communication with the central chamber 180. For example, Figure 24 shows a terminal end plate 132 that comprises seven outlet apertures 131 that are in direct fluid communication with the central chamber(s) 180. In this arrangement, an absence of outlet apertures located in the terminal end plate in direct fluid communication with the gas flow passage opening, means that gas flow within the gas flow passage opening is reflected off the terminal end plate 132 and can bounce back to the inlet end of the muffler, through the inlet aperture(s) 121 and into the central chamber(s) 180.

In yet another form, as shown in Figures 23 and 24, the muffler body is shaped and sized to at least partially fit within a muffler housing 150 and to avoid forming a seal with the muffler housing. In this arrangement, gas outlet apertures 131 is formed by a gap that is defined between the terminal end plate 132 and the muffler housing 150.

In one form, as shown in Figures 13 and 23, the muffler body 110 comprises a core 115 comprising a central chamber 180. The central chamber comprises a blind end at or near the outlet end of the muffler and an inlet aperture 121 to the central chamber 180 at the inlet end of the muffler. A terminal end plate 132 is located at the outlet end of the muffler. In the embodiment shown in Figure 13, the terminal end plate comprises outlet apertures 131 that are spaced equidistant around the terminal end plate 132. The muffler body is configured so that a gap 170a is provided between the outer surface of the core 116 and an internal wall 151 of the muffler housing 150. The gap defines a gas flow passage 170/170a through the muffler. In this form, gas flow enters the central chamber 180 through the inlet aperture 121. The blind end of the chamber 180 causes gas to bounce off inner surfaces of the chamber 180 and then exit the chamber 180 through the inlet aperture 121. Gas then flows along the gas flow passage 170 toward the outlet end of the muffler. In the embodiment of Figure 13, gas may then flow through the outlet openings 131. In the embodiment of Figure 23, an outlet gap may be provided between the terminal end plate 132 and the internal wall 151 of the muffler housing, so that gas can exit the muffler through the outlet gap. The inlet aperture 121 and central chamber 180 comprise a lateral cross-sectional area that is of a different size to the lateral cross-sectional area of the gas flow passage so that gas passing along the gas flow path between the inlet 120 and the outlet 130 is caused to alternately expand and contract or vice versa.

In another form, as shown in Figures 14 to 27, the muffler core 115 comprises at least one central chamber 180 comprising an inlet 121, and also comprises one or more sound attenuating structures 140 that extend along at least a portion of the length of the core 115. In some forms, the sound attenuating structures 140 comprise a curved or angular outwardly facing peripheral surface 141.

The core 115 may be configured so that an outwardly facing peripheral surface 141 of each sound attenuating structure 140 forms a portion of the side surface 117 of the core so that the maximum diameter of the core 115 and the sound attenuating structure(s) 140 is generally equal. In other words, the peripheral surface 141 of each sound attenuating structure 140 is generally flush with the side surface 117 of the core 115, so that the maximum diameter of the muffler core 115 may remain generally consistent along its length. In these forms, the peripheral side surface 117 of the core is at least in part be defined by an outwardly facing peripheral surface 141 of each sound attenuating structure 140.

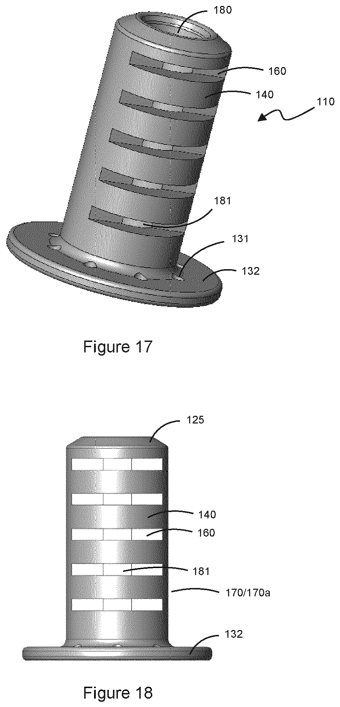

The core 115 may also comprise one or more expansion chambers 160. Generally, each sound attenuating structure 140 is formed by the portions of the muffler body that lie between expansion chambers 160. Where the muffler comprises a series of expansion chambers 160, a sound attenuating structure 140 is provided between adjacent expansion chambers 160 to form a series of sound attenuating structures 140 that extend along at least a portion of the length of the core 115. In some forms, as shown in Figures 14 to 16, the sound attenuating structures 140 extend along only a portion of the core 115. In other forms, as shown in Figures 17 and 18, the sound attenuating structures 140 extend along almost the entire length of the core 115.

The sound attenuating structures may extend around the circumference of the muffler core 115 to form annular discs, or the sound attenuating structures may extend around only a portion of the muffler core to form rib-like members. Figures 14 and 17 show embodiments in which the muffler has two sets of rib-like sound attenuating structures, one set on opposing sides of the core 115. The sound attenuating structures 140 may project laterally from an outside surface of the central chamber 180, which may form a central shaft 114 of the core 115, or from a central chamber structure comprising each central chamber (where the core comprises more than one central chamber). In one form, the muffler core 115 is generally cylindrical and the sound attenuating structures 140 have a curved peripheral surface that is dimensioned to continue the generally cylindrical shape of the core 115.

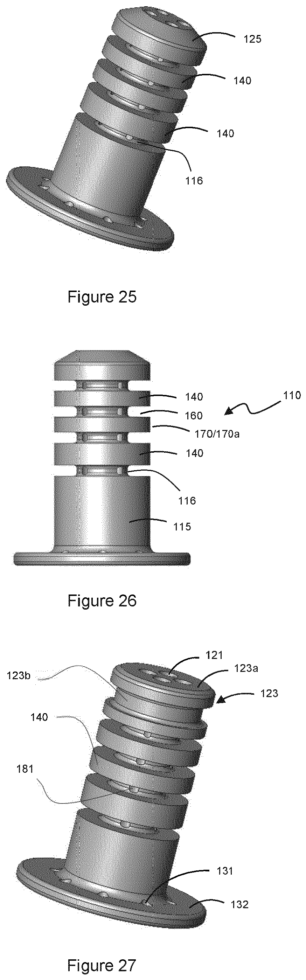

The sound attenuating structures 140 may be of different sizes and/or shapes. In some forms, the muffler comprises a series of sound attenuating structures 140 extending along the length of the muffler. The size of each sound attenuating structure 140, such as the width and/or length of each structure, may increase and/or decrease toward the outlet end of the core 115, as shown in Figures 25 to 27. The sound attenuating structures 140 may each have different sizes from one another. It is possible to vary the distance between adjacent sound attenuating structures by varying the width of the intervening expansion chamber.

An expansion chamber 160 may be provided between adjacent sound attenuating structures 140. Where the muffler body 110 comprises multiple expansion chambers 160, a sound attenuating structure 140 is formed between adjacent chambers 160. The expansion chambers 160 define the size and shape of the sound attenuating structures 140, which may comprise rib-like structures along at least a portion of the length of the core 115.

In one form, as shown in Figure 14 to 27, the muffler body 110 comprises one or more expansion chambers 160 that may be defined by cutout regions that extend along at least a portion of the side surface 117 of the muffler core 115. In some forms, the cutout regions may be provided along almost the entire length of the muffler core 115. The cutout regions may comprise slots or any other suitable shape.

The expansion chambers 160 may be of the same size and shape or the expansion chambers 160 may be of different sizes in shapes. In some forms, the muffler 100 may comprise a series of expansion chambers 160 along at least a portion of its length and the size of the expansion chambers 160, such as the width and/or length of the cutout regions, may increase toward the outlet end of the core 116.

The expansion chambers 160 may lie along a plane that bisects the central longitudinal axis running along the length of the muffler body between the inlet end and the outlet end. For example, the expansion chambers may be perpendicular to the longitudinal direction of flow through the muffler body. In other forms, the expansion chambers 160 may be diagonal to the longitudinal direction of flow through the muffler body.

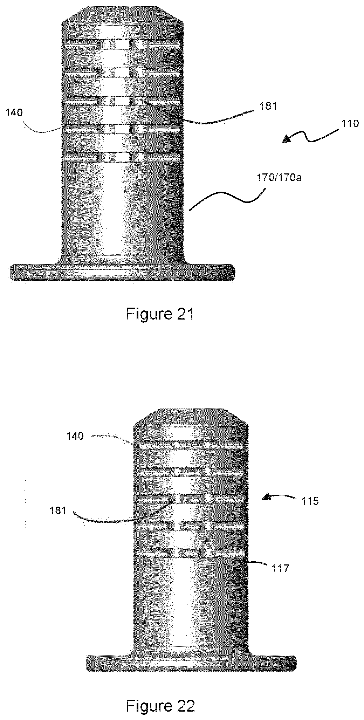

One or more chamber apertures 181 are provided in the side wall defining the central chamber 180. Where multiple apertures 181 are provided in the chamber side wall, the apertures 181 may be the same or different shapes and sizes. In one form, as shown in Figure 22, the chamber apertures increase in size along the length of the muffler and toward the outlet end of the core 115. The chamber apertures 181 may extend radially through the chamber side wall to direct gas outwardly toward the internal wall 151 of the muffler housing 150. However, in some forms the chamber apertures 181 may be configured to direct gas flow in different directions so that the emitted gas flows meet and create interference. It is considered that the interference helps to attenuate the sound of the gas passing through the muffler.

The expansion chamber(s) 160 are located along the core 115 to generally align with the chamber aperture(s) 181 so that the expansion chamber(s) 160, central chamber 180, and chamber aperture(s) 181 are in fluid communication. Because the central chamber 180 is sealed at or near the outlet end, gas flowing into the chamber 180 from the inlet 121 is forced through the chamber apertures 181. Gas passing through the chamber apertures 181 is directed into an expansion chamber 160 of the muffler 100, as shown in Figure 21, 22 and 25 to 27. Each expansion chamber 160 has a lateral cross-sectional area greater than the lateral cross-sectional area of each chamber aperture 181. Gas is therefore contracted under pressure as it passes through one of the chamber apertures 181 and then expands as it enters one of the expansion chambers 160.

The muffler of Figures 13 to 27 may comprise various adaptations to tune the muffler for its desired use and location in a respiratory or a surgical insufflation system.

For example, Figures 13, 14 and 17 show a muffler comprising one inlet aperture 121 leading to one central chamber 180 and Figure 20 shows a muffler with four inlet apertures 121 leading to four central chambers 180. Where the muffler comprises multiple central chambers 180, such as in the embodiments of Figures 19 to 22, the chambers 180 may be equal sized or the sizes of the chambers may vary. In some forms, as shown in Figures 25 to 27, the central chambers 180 may be joined together to form a central shaft or structure from which the sound attenuating structures 140 project laterally. In another form, the sound attenuating may project laterally from multiple central chambers that are not otherwise joined together.

In some forms, as shown in Figures 14 to 26, the inlet 120 of the muffler may comprise a bevelled/chamfered surface 125 that angles toward the side surface 117 of the core. For example, the inlet end of the muffler may comprise a bevelled peripheral edge or surface 125. In this arrangement, the bevelled surface 125 forms a flow directing element that directs gas flow towards one or more gas flow passage openings 170 comprising a gap 170a formed between the core and sound attenuating structure(s) 140 of the muffler and the internal wall 151 of the muffler housing. Therefore, gas flow may enter the muffler through one or more inlet apertures 121 and into the central chamber(s) 180, and/or gas flow may be directed to flow through one or more gas flow passage openings 170/170a. In some forms, the muffler body 110 may seal against the muffler housing 150 so that gas can only enter the muffler via the inlet aperture(s) 121. In these forms, the bevelled surface 125 may act as an aid to ensure sealing with the muffler housing 150. In other forms, the muffler body 110 may be shaped and sized to provide at least one inlet gap between the muffler body 110 and the internal wall of the muffler housing 150. In this form, gas flow may enter the muffler through the inlet aperture(s) 121 and through one or more inlet gaps formed between the inlet end of the muffler body 110 and the muffler housing 150.

In yet another form, the muffler comprises an inlet that comprises a gap formed between the outer peripheral surface 117 of the muffler core 115 and the internal wall 151 of the muffler housing. In this form, the inlet end of the muffler may comprise an outwardly facing, bevelled surface 125 that directs gas flow outwardly and toward the inlet gap between the core 115 and housing wall 151. In some forms, the muffler body 110 comprises a locating element to locate the body 110 within the muffler housing 150 so as to form a consistent gap between the side surface of the muffler core 115 and the internal wall 151 of the housing 150. In some forms, at least a portion of the muffler body may comprise a bevelled surface 125, which may comprise a locating element to locate the body 110 within the muffler housing 150.

In any or all of the embodiments of the invention, the muffler body 110 may comprise a locating element to locate the body 110 concentrically within the muffler housing 150. In some forms, the muffler body is located within the muffler housing to provide a substantially consistently sized gap between the outer surface 116 of the muffler core 115 and the internal wall 151 of the housing. In this arrangement, the gap may provide a gas flow passage 170/170a having a generally consistent lateral cross-sectional area.

The locating element may be any suitable component or structure, such as two or more, arms that extend radially from the muffler body and that are configured to contact the internal wall 151 of the muffler housing. In some forms, the locating element comprises three or more radially extending arms that are located at or near the inlet end portion of the muffler body 110. In another form, the locating element may comprise a sealing element, as described above, such as an annular seal, o-ring, interference seal, adhesive, or the like that contacts the internal wall 151 of the muffler housing to locate the muffler body 110 within the housing 150.

The muffler 100, in the embodiments of the invention may therefore comprises a tortuous gas flow path around the central longitudinal axis of the muffler. For example, the sound attenuating structure(s) 140 may be configured to provide a tortuous gas flow path between the inlet 120 and outlet 130 of the muffler and/or the sound attenuating structures 140 may be configured to provide a gas flow path having variable cross-sectional areas along the length of the muffler 100.

In some forms, the muffler can be tuned to create a muffler that provides desired sound attenuating characteristics. For example, the dimensions and size of the muffler, the size of the central chamber 180; the size and number of chamber apertures 181, sound attenuating structures 140, and expansion chambers 160; and the distance between sound attenuating structures 140 may be altered/tuned to provide the muffler with a desired performance. The primary considerations when tuning the muffler are the pressure drop across the muffler as well as the level of sound attenuation that wants to be achieved. The muffler of Figure 17, for example, comprises expansion chambers that a wider than those of the muffler shown in Figure 19. Similarly, the muffler of Figure 22 comprises chamber apertures of different sizes, compared to those in the muffler of Figure 21. These are examples of mufflers that have been configured to provide different performance characteristics. In some forms, the muffler may be configured/tuned in a certain way to make the muffler easy to manufacture. This may be particularly important if the muffler is moulded.

The sound attenuating structures 140 may be sized and shaped so that the peripheral edge 140a of each structure has a width/diameter that is less than the internal width/diameter of the muffler housing 150. An annular gap is therefore formed between the peripheral edge of each sound attenuating structure 140 and the internal wall of the muffler housing, and between the side surface 116 of the muffler shaft or core 115 and the internal wall 151 of the housing 150. The annular gap forms a gas flow passage 170 having a smaller cross-section than the cross-section of each expansion chamber 160. The gas flow passage forms a portion of the gas flow path that extends between the inlet 120 and outlet 130 of the muffler 100.

Gas flow within mufflers comprising one or more central chambers 180 with chamber apertures 181 to direct gas into multiple expansion chambers 160, and gas flow within mufflers that direct gas through one or more central chambers 180 and through a gap 170 provided between the muffler core 115 and muffler housing 150 may follow a changeable, tortuous gas flow path that regularly changes direction, especially as gas reflects off surfaces of the muffler or interferes with other gas flows through the muffler. The nature of the central chamber(s) 180, chamber apertures 181, expansion chambers 160, gas flow passage openings/gaps 170 and sound attenuating structures 140 means that gas flow will move in many directions, creating a tortuous gas flow path through the muffler 100. This may increase sound cancellation/dampening due to sound waves interfering with each other and with surfaces of the muffler.

In use, gas passing into the muffler 100 is forced to follow a gas flow path that passes through or around each sound attenuating structure 140. For example, after entering the muffler through the inlet 120, gas may pass through one or more gas flow passage openings 170b, 170c formed in a sound attenuating structure 140 located closest to the inlet aperture exit opening(s) 182. Alternatively or additionally, gas may pass through a gas flow passage opening/gap 170a formed between the sound attenuating structure 140 and the internal wall 151 of the muffler housing 150. In some forms, the gas flow passage gap closest to the inlet end of the muffler forms an inlet as this is the first entry point at which gas enters the muffler. Gas passing through the gas flow passage opening(s) 170 undergoes a pressure increase due to the small cross-sectional area of the opening(s) 170.

The muffler therefore provides a gas flow path comprising a variable cross-section to allow the gas to expand and contract at different portions of the gas flow path. That is, the gas flow moves alternatingly between a series of contraction portions (the gaps/apertures of the gas flow passages) and expansion portions (the expansion chambers) along the gas flow path, causing the gas flow to alternately vary between smaller and larger cross-sectional areas from the muffler inlet 120 to the muffler outlet 130.

For example, as shown in Figures 28 and 29, the internal wall 151 of the muffler housing 150 is spaced from the central longitudinal axis 500 of the muffler at a distance R1 and at a distance D1 from a portion of at least one sound attenuating structure 140, such as an outer peripheral surface 141 of the sound attenuating structure. The distance D1 is greater than zero (D1 > 0) to form a gap between the sound attenuating structure 140 and the internal wall 151. The gap forms a contraction portion of the gas flow path. The internal wall 151 may also be located at a distance D2 from the shaft, such as from an outer peripheral surface 116 of the shaft. The distance D2 is greater than the distance D1 (D2 > D1) to form an expansion chamber between the shaft and the internal wall 151. The expansion chamber forms an expansion portion of the gas flow path. The contraction portion of the gas flow path has a smaller lateral cross-sectional area than the expansion portion of the gas flow path, so that gas passing through the muffler passes between alternating expansion and contraction portions of the gas flow path to help attenuate sound from the gas flow.

Additionally or alternatively, the muffler 100 may be configured to provide a tortuous gas flow path between the muffler inlet 120 and the muffler outlet 130, as described above, to help cancel/dampen the sound of gas passing through the muffler.

In some forms, the distance from the muffler inlet 120 to the muffler outlet 130 corresponds to a sound frequency/wavelength to be reduced, removed or dampened by the muffler 100. Typical sound frequencies from a gas flow source for a respiratory support system are between 10 to 20 kHz. In one form, the distance from the inlet 120 to the outlet 130 is at least 20mm, and optionally 30mm. In another form, the distance from the inlet 120 to the outlet 130 is between about 20mm to about 100mm inclusive.

The muffler 100 provides sound attenuation by causing the gas flow to repeatedly contract and expand as the gas moves between smaller and larger cross-sectional areas of the gas flow path. Sound attenuation may also result from sound waves reflecting off surfaces of the muffler, such as off surfaces of a central shaft 114 of the muffler, one or more central chambers 180 of the muffler and/or off surfaces of the sound attenuating structures 140, to interfere with other sound waves.

The muffler 100 may be made of any suitable materials, such as plastic or metal for example. Typically, the muffler is moulded in shape.

The muffler 100 may comprise one or more sound absorbing materials. The sound absorbing material(s) may form a surface of the muffler or at least a portion of the muffler may be made from one or more sound absorbing materials. For example, the muffler body, and/or one or more sound attenuating structures, and/or the muffler housing may comprise one or more sound absorbing materials. In one form, the sound absorbing material(s) may be located on one or more surfaces of the muffler body, such as a covering layer or coating, or the muffler body may be formed to include one or more materials that provide a sound absorbing outer surface. Additionally or alternatively, a layer or coating of sound absorbing materials may be applied to at least one surface of one of more sound attenuating structure or to at least one internal surface of the muffler housing. Examples of suitable sound absorbing materials include filter material, woven or knitted fabric, polyurethane foam, fibrous materials, sintered materials or other fibres that may be porous, natural or synthetic. In some forms, one or more of these materials may be placed at the inlet or outlet of the muffler or in between sound attenuating structures, or anywhere else along the gas flow path. Where the muffler comprises one or more central chambers, the central chamber(s) may comprise a sintered plastic/metal disc or rod to help absorb sound. The disc may also comprise a suitable sound absorbing material, which may include any of the suitable materials listed above.

Additionally or alternatively, the muffler 100 may be configured to comprise other features to attenuate sound. For example, one or more sound attenuating structures 140 may comprise chamfered edges. In another example, surface indentations, patterns or surface finishes (to increase the roughness of a surface) may be applied to one or more surfaces of the muffler 100 that lie along the gas flow path. Increasing the roughness of a surface may cause sound waves to bounce of surfaces, which may increase interference with other sound waves and increase sound attenuation.

The versatility of the muffler 100 disclosed herein allows the muffler to be used anywhere along the gas flow path of a respiratory support system 10. For example, in some forms, the muffler 100 is directly connectable to a gas flow source 12, such as a wall flow source of breathing gas or a blower. For example, the muffler inlet may comprise an engagement mechanism to engage a gas flow source. The engagement mechanism may be of any suitable form. In one form, the engagement mechanism comprises screw threads for threading the muffler to the gas flow source.

In some forms, the muffler 100 may be connectable to a humidifier 17, so as to be in fluid communication with the humidifier. For example, the muffler 100 may comprise an engagement mechanism to engage a humidifier 17. The engagement mechanism may be of any suitable form. In one form, the engagement mechanism comprises screw threads for threading the muffler to the humidifier. The engagement mechanism may be provided at the inlet 120 or the outlet 130 of the muffler.

In some forms, the muffler 100 is configured to be placed along a gas flow path of a pressure regulating device or pressure relief valve 200, such as a flow compensated pressure relief valve, as shown in Figure 9. Optionally, the pressure relief valve 200 may be a valve having features described in WO/2018/033863.

In some forms, the muffler 100 may be insertable within, or configured to be directly coupled to, an inlet 210 or an outlet 220 of a pressure relief valve 200, as shown in Figure 9. Alternatively, the muffler 100 may be integrally formed with the pressure relief valve 200. In other forms, the muffler 100 may be configured to be coupled to a pressure relief valve 200 in a respiratory system 10, either upstream or downstream from the pressure relief valve, via a conduit. In some forms, the muffler 100 may be inserted within the conduit. In other forms, the muffler may be coupled to the conduit.

In another form, as shown in Figure 11, the muffler may be insertable within or coupled to a spring plunger pressure relief valve. For example, the muffler 100 may be located within the inlet 210 or the outlet 220 of the pressure relief valve 200, the muffler 100 may be coupled to the inlet 210 or the outlet 220 of the pressure relief valve 200, or the muffler 100 may be in fluid communication with the pressure relief valve 200 via a conduit. The muffler may be connected to the conduit so as to be in fluid communication with the conduit, or the muffler may be located along a gas flow path within the conduit.

One form of pressure relief valve according to the invention, that is used with the muffler as disclosed herein in a respiratory system is shown in Figure 9 and comprises an inlet 210 and an outlet chamber 205 with an outlet 220. The inlet 210 is in fluid communication with the outlet chamber 205. A valve seat 230 is located between the inlet 210 and the outlet 220. A valve member 240, optionally comprising an elastomeric membrane, may be suspended across the outlet chamber 205. The membrane 240 may be stretched or tensioned over the valve seat 230 so that tension in the membrane 240 causes the membrane to be biased against the valve seat 230 to form a seal with the valve seat and close a flow path from the inlet 210 to the outlet chamber 205. The membrane 240 is further configured to be displaced from the valve seat 230 by an inlet pressure at the inlet 210 increasing above a pressure threshold to allow a flow of gases from the gas flow path between the inlet 210 to the outlet 220 to vent from the valve via the outlet chamber 205. The pressure relief valve 200 may have a sensing element that compensates for the rate of flow through the valve 200 by biasing the valve membrane 240 on the valve seat 230 depending on the gas flow rate.

In some forms, the muffler 100 may be provided within the housing of a pressure relief valve 200. In one form, the muffler comprises an insert to be inserted within a pressure relief valve 200 or a conduit. For example, a pressure relief valve may comprise a muffler inserted within the valve inlet 210 or the valve outlet 220. Optionally, the valve 200 may comprise a first muffler inserted within the valve inlet and a second muffler inserted within the valve outlet. In another form, the muffler 100 may be integrally formed with the inlet 210 and/or outlet 220 of the pressure relief valve 200. In these arrangements, the internal wall of the valve inlet or outlet or the conduit, as the case may be, may form a housing for the muffler. For example, where the sound attenuating structure(s) of the muffler are configured to define a gap between a distal edge of the structure(s) and an internal wall, the gap will be defined between the distal edge of the structure(s) and the internal wall of the inlet or outlet within which the muffler is located.

In one form, the muffler 100 may be attached to the inlet 210 of the pressure relief valve 200 and may comprise an elongate channel portion or other gas conduit to provide fluid communication with a gas flow source 12. The muffler 100 or muffler and valve assembly 100-200 may be removably coupled to the gas source 12. For example, the muffler 100 or pressure relief valve 200 may comprise a threaded connection or another suitable connection system, such as an interference connection or friction fit connection, to connect to the gas source 12.

The invention relates to a pressure relief valve 200 that comprises a muffler 100. The valve inlet 210 of the pressure relief valve 200 comprises an engagement mechanism to couple the pressure relief valve 200 to a gas flow source 12, such as wall flow source of breathing gas flow or a blower. The engagement mechanism may be any suitable form of engagement. In some forms, the engagement mechanism comprises screw threads to thread the pressure relief valve directly onto the gas flow source or onto a conduit that is connected to the gas flow source to provide fluid communication between the pressure relief valve and the gas flow source. In another form, the engagement mechanism may comprise a friction fit between the muffler and the pressure relief valve. For example, the pressure relief valve may comprise a tapering internal surface that contacts an external surface of the muffler body or muffler housing and holds the muffler in engagement with the pressure relief valve through frictional engagement. In yet another form, the engagement mechanism may comprise an adaptor comprising a body comprising an inlet and an outlet. The inlet comprises a first diameter and the outlet comprises a second diameter. The first diameter may be smaller than, larger than or generally equal to the second diameter. In some forms, the adaptor is configured to connect the inlet or outlet of the pressure relief valve 200 to the muffler 100.

According to the invention, the valve outlet 220 is connectable to a humidifier 17 via a gas conduit to provide fluid communication between the pressure relief valve and the humidifier.

When used in a respiratory support system, the muffler 100 of the embodiments described herein, has been found to attenuate sound emitted by the system to about 50dBA or less, under normal use gas flows, which is considered to be an appropriate sound level for hospitals, surgical theatres, and at home. Gases from a compressed gas source at high flow rates though a needle valve with no muffler may typically emit a sound that is approximately 70 dBA or more. The muffler of the embodiments described herein attenuates sound to a suitable level of approximately 50 dBA or less.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to".

Reference to any prior art in this specification is not, and should not be taken as, an acknowledgement or any form of suggestion that that prior art forms part of the common general knowledge in the field of endeavour in any country in the world.

Where, in the foregoing description reference has been made to integers or components having known equivalents thereof, those integers are herein incorporated as if individually set forth.

Preferred embodiments of the invention have been described by way of example only. For example, the dimensions mentioned above are provided as examples only and may vary in mufflers and pressure relief valves of different sizes or constructed from different materials to account for different material properties. The invention is defined by the claims which follow.

## Claims

1. A pressure relief valve (200) for use in respiratory support systems, comprising:
a valve inlet (210) and a valve outlet (220); and
a respiratory system muffler (100) located along a gas flow path of the pressure relief valve (200), the gas flow path extending between the valve inlet and the valve outlet; wherein the muffler (100) comprises a tortuous gas flow path to attenuate sound; wherein the valve inlet (210) comprises an engagement mechanism to couple the pressure relief valve to a gas flow source (12);
wherein the valve outlet (220) is connectable to a humidifier (17) via a gas conduit to provide fluid communication between the pressure relief valve and the humidifier; and
wherein the pressure relief valve (200) comprises a flow compensated pressure relief valve and is configured to allow gases from a flow of gases along the gas flow path to vent above a pressure threshold.

2. The pressure relief valve of claim 1, wherein the muffler (100) is located at the valve inlet (210) or the valve outlet (220) or both; or wherein the muffler (100) is inserted within the valve inlet (210) or the valve outlet (220).

3. The pressure relief valve of any one of claims 1 to 2, wherein the tortuous flow path comprises different cross-sectional gas flow areas.

4. The pressure relief valve of any one of claims 1 to 3, wherein the tortuous flow path comprises at least one contraction portion where the gas flow is caused to contract and at least one expansion portion where the gas flow is caused to expand.

5. The pressure relief valve of any one of claims 1 to 4, wherein the muffler (100) comprises a housing (150), a muffler inlet (120), a muffler outlet (130), and a sound attenuating structure that defines a gap (170a) between a peripheral surface (141) of the sound attenuating structure (140) and an internal wall (151) of the housing (150), wherein the gap (170a) forms a portion of the gas flow path; and wherein the muffler (100) comprises one or more sound absorbing materials.

6. The pressure relief valve of claim 5, wherein the sound attenuating structure (140) comprises a laterally extending projection that extends towards the internal wall (151) of the housing (150).

7. The pressure relief valve according to claim 6, wherein the laterally extending projection terminates proximate to the internal wall (151) of the housing (150) and at least a portion of the gas flow path is defined by a gap (170a) formed between the peripheral surface of the projection and the internal wall (151) of the housing (150).

8. The pressure relief valve according to claim 7, wherein the gap (170a) is about 0.5 mm wide or less; or
the gap (170a) has a width that is between about 0.1 mm to about 0.5 mm inclusive; or
the gap (170a) is about 0.25 mm wide.

9. The pressure relief valve according to any one of claims 1 to 8, wherein the muffler (100) comprises two or more sound attenuating structures (140).

10. The pressure relief valve according to claim 9, wherein an expansion chamber (160) is defined between two adjacent ones of the sound attenuating structures.

11. The pressure relief valve according to claim 9 or 10, wherein a constant or variable distance is provided between the sound attenuating structures (140); and/or wherein each sound attenuating structure (140) has the same thickness, or at least one of the sound attenuating structures (140) has a different thickness to one or more others of the sound attenuating structures (140).

12. The pressure relief valve according to any one of claims 10 to 11, wherein the muffler inlet (120) comprises a flow directing element (122) that directs gas flow to the sound attenuating structure(s) (140) or, when depending on claim 10, to the expansion chamber (160).

13. The pressure relief valve according to any one of claims 1 to 12, wherein the muffler inlet (120) comprises one or more inlet apertures (121).

14. The pressure relief valve according to any one of claims 1 to 13, wherein the muffler (100) comprises a terminal end plate (132) on which the muffler outlet (130) is located and wherein the muffler outlet (130) comprises one or more outlet apertures (131).

15. The pressure relief valve according to any one of claims 1 to 14, wherein the distance from the muffler inlet (120) to the muffler outlet (130) corresponds to a sound frequency to be reduced, removed or dampened by the muffler.

16. The pressure relief valve according to any one of claims 1 to 15, wherein the muffler (100) comprises an outlet end portion, on which the muffler outlet (130) is located, and wherein the outlet end portion comprises a sealing element (300) adapted to seal against a surface of the pressure relief valve (200), optionally wherein the sealing element (300) is located on an external surface of the muffler housing (150).

17. The pressure relief valve according to any one of claims 1 to 15, wherein the pressure relief valve (200) further comprises an outlet chamber (205) with a vent outlet for pressure relief.

## Patentansprüche

1. Druckentlastungsventil (200) zur Verwendung in Atemunterstützungssystemen, umfassend:
einen Ventileinlass (210) und einen Ventilauslass (220); und
einen Schalldämpfer (100) für ein Atemsystem, der entlang eines Gasströmungswegs des Druckentlastungsventils (200) angeordnet ist, wobei sich der Gasströmungsweg zwischen dem Ventileinlass und dem Ventilauslass erstreckt;
wobei der Schalldämpfer (100) einen gewundenen Gasströmungsweg umfasst, um Schall zu dämpfen;
wobei der Ventileinlass (210) einen Eingriffsmechanismus umfasst, um das Druckentlastungsventil mit einer Gasströmungsquelle (12) zu koppeln;
wobei der Ventilauslass (220) über eine Gasleitung mit einem Befeuchter (17) verbindbar ist, um eine Fluidverbindung zwischen dem Druckentlastungsventil und dem Befeuchter bereitzustellen; und
wobei das Druckentlastungsventil (200) ein strömungskompensiertes Druckentlastungsventil umfasst und dazu ausgelegt ist, ein Entweichen von Gasen aus einem entlang des Gasströmungswegs verlaufenden Gasstrom oberhalb eines Druckschwellenwerts zu ermöglichen.

2. Druckentlastungsventil nach Anspruch 1, wobei der Schalldämpfer (100) am Ventileinlass (210) oder am Ventilauslass (220) oder an beiden angeordnet ist; oder wobei der Schalldämpfer (100) innerhalb des Ventileinlasses (210) oder des Ventilauslasses (220) eingesetzt ist.

3. Druckentlastungsventil nach einem der Ansprüche 1 bis 2, wobei der gewundene Strömungsweg unterschiedliche Gasströmungsquerschnittsflächen umfasst.

4. Druckentlastungsventil nach einem der Ansprüche 1 bis 3, wobei der gewundene Strömungsweg mindestens einen Verengungsabschnitt umfasst, in dem eine Verengung der Gasströmung bewirkt wird, und mindestens einen Erweiterungsabschnitt, in dem eine Erweiterung der Gasströmung bewirkt wird.

5. Druckentlastungsventil nach einem der Ansprüche 1 bis 4, wobei der Schalldämpfer (100) ein Gehäuse (150), einen Schalldämpfereinlass (120), einen Schalldämpferauslass (130) und eine Schalldämpfungsstruktur umfasst, die einen Spalt (170a) zwischen einer Umfangsfläche (141) der Schalldämpfungsstruktur (140) und einer Innenwand (151) des Gehäuses (150) definiert, wobei der Spalt (170a) einen Abschnitt des Gasströmungswegs bildet; und wobei der Schalldämpfer (100) ein oder mehrere schallabsorbierende Materialien umfasst.

6. Druckentlastungsventil nach Anspruch 5, wobei die Schalldämpfungsstruktur (140) einen sich seitlich erstreckenden Vorsprung umfasst, der sich in Richtung der Innenwand (151) des Gehäuses (150) erstreckt.

7. Druckentlastungsventil nach Anspruch 6, wobei der sich seitlich erstreckende Vorsprung nahe der Innenwand (151) des Gehäuses (150) endet und mindestens ein Abschnitt des Gasströmungswegs durch einen Spalt (170a) definiert ist, der zwischen der Umfangsfläche des Vorsprungs und der Innenwand (151) des Gehäuses (150) gebildet ist.

8. Druckentlastungsventil nach Anspruch 7, wobei der Spalt (170a) eine Breite von etwa 0,5 mm oder weniger aufweist; oder
der Spalt (170a) eine Breite zwischen etwa 0,1 mm und etwa 0,5 mm, einschließlich der Grenzwerte, aufweist; oder
der Spalt (170a) eine Breite von etwa 0,25 mm aufweist.

9. Druckentlastungsventil nach einem der Ansprüche 1 bis 8, wobei der Schalldämpfer (100) zwei oder mehr Schalldämpfungsstrukturen (140) umfasst.

10. Druckentlastungsventil nach Anspruch 9, wobei zwischen zwei benachbarten Schalldämpfungsstrukturen eine Expansionskammer (160) definiert ist.

11. Druckentlastungsventil nach Anspruch 9 oder 10, wobei zwischen den Schalldämpfungsstrukturen (140) ein konstanter oder variabler Abstand vorgesehen ist; und/oder
wobei jede Schalldämpfungsstruktur (140) die gleiche Dicke aufweist oder mindestens eine der Schalldämpfungsstrukturen (140) eine von einer oder mehreren der anderen Schalldämpfungsstrukturen (140) abweichende Dicke aufweist.

12. Druckentlastungsventil nach einem der Ansprüche 10 bis 11, wobei der Schalldämpfereinlass (120) ein Strömungsleitelement (122) umfasst, das die Gasströmung zu der Schalldämpfungsstruktur beziehungsweise den Schalldämpfungsstrukturen (140) oder, bei Rückbezug auf Anspruch 10, zu der Expansionskammer (160) lenkt.

13. Druckentlastungsventil nach einem der Ansprüche 1 bis 12, wobei der Schalldämpfereinlass (120) eine oder mehrere Einlassöffnungen (121) umfasst.

14. Druckentlastungsventil nach einem der Ansprüche 1 bis 13, wobei der Schalldämpfer (100) eine abschließende Endplatte (132) umfasst, auf der der Schalldämpferauslass (130) angeordnet ist, und wobei der Schalldämpferauslass (130) eine oder mehrere Auslassöffnungen (131) umfasst.

15. Druckentlastungsventil nach einem der Ansprüche 1 bis 14, wobei der Abstand vom Schalldämpfereinlass (120) zum Schalldämpferauslass (130) einer Schallfrequenz entspricht, die durch den Schalldämpfer reduziert, beseitigt oder gedämpft werden soll.

16. Druckentlastungsventil nach einem der Ansprüche 1 bis 15, wobei der Schalldämpfer (100) einen Auslassendabschnitt umfasst, an dem der Schalldämpferauslass (130) angeordnet ist, und wobei der Auslassendabschnitt ein Dichtungselement (300) umfasst, das dazu ausgelegt ist, gegenüber einer Oberfläche des Druckentlastungsventils (200) abzudichten, wobei das Dichtungselement (300) optional an einer Außenfläche des Schalldämpfergehäuses (150) angeordnet ist.

17. Druckentlastungsventil nach einem der Ansprüche 1 bis 15, wobei das Druckentlastungsventil (200) ferner eine Auslasskammer (205) mit einem Entlüftungsauslass zur Druckentlastung umfasst.

## Revendications

1. Vanne de décompression (200) pour une utilisation dans des systèmes d'assistance respiratoire, comprenant :
une entrée de vanne (210) et une sortie de vanne (220) ; et
un silencieux de système respiratoire (100) situé le long d'un chemin d'écoulement de gaz de la vanne de décompression (200), le chemin d'écoulement de gaz s'étendant entre l'entrée de vanne et la sortie de vanne ;
dans laquelle le silencieux (100) comprend un chemin d'écoulement de gaz tortueux pour atténuer le son ;
dans laquelle l'entrée de vanne (210) comprend un mécanisme de mise en prise pour coupler la vanne de décompression à une source d'écoulement de gaz (12) ;
dans laquelle la sortie de vanne (220) est apte à être raccordée à un humidificateur (17) via un conduit de gaz pour fournir une communication fluidique entre la vanne de décompression et l'humidificateur ; et
dans laquelle la vanne de décompression (200) comprend une vanne de décompression à compensation d'écoulement et est configurée pour permettre à des gaz provenant d'un écoulement de gaz le long du chemin d'écoulement de gaz d'être évacués au-dessus d'un seuil de pression.

2. Vanne de décompression selon la revendication 1, dans laquelle le silencieux (100) est situé au niveau de l'entrée de vanne (210) ou de la sortie de vanne (220) ou des deux ; ou dans laquelle le silencieux (100) est inséré au sein de l'entrée de vanne (210) ou de la sortie de vanne (220).

3. Vanne de décompression selon l'une quelconque des revendications 1 à 2, dans laquelle le chemin d'écoulement tortueux comprend des zones d'écoulement de gaz à sections transversales différentes.

4. Vanne de décompression selon l'une quelconque des revendications 1 à 3, dans laquelle le chemin d'écoulement tortueux comprend au moins une partie de contraction où l'écoulement de gaz est amené à se contracter et au moins une partie de dilatation où l'écoulement de gaz est amené à se dilater.

5. Vanne de décompression selon l'une quelconque des revendications 1 à 4, dans laquelle le silencieux (100) comprend un boîtier (150), une entrée de silencieux (120), une sortie de silencieux (130) et une structure d'atténuation de son qui définit un espace (170a) entre une surface périphérique (141) de la structure d'atténuation de son (140) et une paroi interne (151) du boîtier (150), dans laquelle l'espace (170a) forme une partie du chemin d'écoulement de gaz ; et dans laquelle le silencieux (100) comprend un ou plusieurs matériaux d'absorption de son.

6. Vanne de décompression selon la revendication 5, dans laquelle la structure d'atténuation de son (140) comprend une saillie s'étendant latéralement qui s'étend vers la paroi interne (151) du boîtier (150).

7. Vanne de décompression selon la revendication 6, dans laquelle la saillie s'étendant latéralement se termine à proximité de la paroi interne (151) du boîtier (150) et au moins une partie du chemin d'écoulement de gaz est définie par un espace (170a) formé entre la surface périphérique de la saillie et la paroi interne (151) du boîtier (150).

8. Vanne de décompression selon la revendication 7, dans laquelle l'espace (170a) mesure environ 0,5 mm de large ou moins ; ou
l'espace (170a) possède une largeur qui mesure entre environ 0,1 mm et environ 0,5 mm inclus ; ou
l'espace (170a) mesure environ 0,25 mm de large.

9. Vanne de décompression selon l'une quelconque des revendications 1 à 8, dans laquelle le silencieux (100) comprend deux structures d'atténuation de son (140) ou plus.

10. Vanne de décompression selon la revendication 9, dans laquelle une chambre de dilatation (160) est définie entre deux structures adjacentes parmi les structures d'atténuation de son.

11. Vanne de décompression selon la revendication 9 ou 10, dans laquelle une distance constante ou variable est ménagée entre les structures d'atténuation de son (140) ; et/ou
dans laquelle chaque structure d'atténuation de son (140) possède la même épaisseur, ou au moins l'une des structures d'atténuation de son (140) possède une épaisseur différente d'une ou de plusieurs autres structures parmi les structures d'atténuation de son (140).

12. Vanne de décompression selon l'une quelconque des revendications 10 à 11, dans laquelle l'entrée de silencieux (120) comprend un élément d'orientation d'écoulement (122) qui oriente l'écoulement de gaz vers la ou les structure(s) d'atténuation de son (140) ou, lorsqu'elle dépend de la revendication 10, vers la chambre de dilatation (160).

13. Vanne de décompression selon l'une quelconque des revendications 1 à 12, dans laquelle l'entrée de silencieux (120) comprend une ou plusieurs ouvertures d'entrée (121).

14. Vanne de décompression selon l'une quelconque des revendications 1 à 13, dans laquelle le silencieux (100) comprend une plaque d'extrémité terminale (132) sur laquelle la sortie de silencieux (130) est située et dans laquelle la sortie de silencieux (130) comprend une ou plusieurs ouvertures de sortie (131).

15. Vanne de décompression selon l'une quelconque des revendications 1 à 14, dans laquelle la distance de l'entrée de silencieux (120) à la sortie de silencieux (130) correspond à une fréquence sonore devant être réduite, éliminée ou atténuée par le silencieux.

16. Vanne de décompression selon l'une quelconque des revendications 1 à 15, dans laquelle le silencieux (100) comprend une partie d'extrémité de sortie, sur laquelle la sortie de silencieux (130) est située, et dans laquelle la partie d'extrémité de sortie comprend un élément d'étanchéité (300) adapté pour assurer l'étanchéité contre une surface de la vanne de décompression (200), facultativement dans laquelle l'élément d'étanchéité (300) est situé sur une surface externe du boîtier de silencieux (150).

17. Vanne de décompression selon l'une quelconque des revendications 1 à 15, dans laquelle la vanne de décompression (200) comprend en outre une chambre de sortie (205) dotée d'une sortie d'évacuation pour une décompression.
